# EUROPEAN PATENT APPLICATION

(11) **EP 4 431 528 A1**
(43) Date of publication of application: **18.09.2024**
(21) Application number: 21961862.6
(22) Date of filing: 29.10.2021
(51) Int. Cl.: C07K 16/42, C07K 16/28, C12N 15/63, A61P 37/08, A61K 47/18, A61K 39/395, A61K 39/00, A61P 11/06

(54) **ISOLATED ANTIGEN BINDING PROTEIN AND USE THEREOF**

(71) Applicant: Longbio Pharma (Suzhou) Co., Ltd., Shanghai 201203 (CN)
(72) Inventor: SUN, Nai-Chau, Shanghai 201203 (CN); SUN, Chow-Rou-Yun, Shanghai 201203 (CN); MA, Haili, Shanghai 201203 (CN); LIU, Yunhua, Shanghai 201203 (CN); YANG, Hongzhou, Shanghai 201203 (CN); GUO, Ruowen, Shanghai 201203 (CN); GAO, Qi, Shanghai 201203 (CN); LIU, Heng, Shanghai 201203 (CN)
(74) Representative: AWA Denmark A/S
(86) International application number: PCT/CN2021/127316
(87) International publication number: WO 2023/070502

(57) **Abstract**

The present application relates to an antigen binding protein comprising at least one CDR of a heavy chain variable region and at least one CDR of a light chain variable region, wherein said heavy chain variable region comprises an amino acid sequence as set forth in any one of SEQ ID NOs: 73 or 74, and the light chain variable region comprises an amino acid sequence as set forth in any one of SEQ ID NOs: 75 or 76.

## Description

### Field of the Invention

The present application relates to the field of biomedicine, and particularly to an antigen binding protein and use thereof.

### Background of the Invention

Allergies (including atopies) and other hypersensitivity disorders are inappropriate or undue immune responses to foreign antigens. The inappropriate immune responses include those misdirected immune responses against components of the body itself, leading to autoimmune diseases. Allergic diseases, also known as hypersensitivity disorders, rank as the sixth most prevalent chronic disease in the world. These diseases affect various tissues and organs within the body, causing a range of discomforting symptoms upon onset, which seriously reduce the quality of life and may even pose life-threatening risks. For example, allergic reactions, allergic rhinitis, asthma, atopic dermatitis, food allergies, and urticaria approximately 20% of the population in many countries, and the prevalence of these diseases is gradually increasing (Wuthrich B., Int. Arch. Allergy AppI. Tmmunol., 90, pp3_10,1989). Allergic diseases can broadly be categorized into IgE-mediated and non-IgE-mediated, with the majority being hypersensitivity reactions mediated by immunoglobulin E (IgE). The high-affinity receptor for IgE (FcεRI) is critical in mediating allergic manifestations. Apart from mast cells and basophils, FcεRI is also found on many other cell types including eosinophils and platelets, and antigen-presenting cells such as monocytes and dendritic cells. Given the crucial role of IgE in mediating a majority of allergic reactions, it is essential to develop therapeutic strategies for controlling IgE levels in allergic diseases.

### Summary of the Invention

The present application provides an antigen binding protein, having one or more of the following advantages: 1) a good stability, a high druggability, and more conducive to industrial production; 2) an enhanced affinity to neonatal Fc receptors (FcRn), a longer half life, and capable of achieving the purpose of prolonging the dosing period.

In some embodiments, the antigen binding protein of the present application includes at least one complementarity determining region (CDR) of a heavy chain variable region (VH) and at least one complementarity determining region (CDR) of a light chain variable region (VL), the heavy chain variable region includes an amino acid sequence as set forth in any one of SEQ ID NOs: 73 or 74, and the light chain variable region includes an amino acid sequence as set forth in any one of SEQ ID NOs: 75 or 76.

In some embodiments, the antigen binding protein of the present application has one or more of the following properties:
1) capable of binding to IgE at a KD value of about 2.4×10⁻⁹ M or below;
2) capable of inhibiting the binding of IgE to its receptors FcεRIa and/or FcεRII.

In some embodiments, the antigen binding protein of the present application binds to human IgE.

In some embodiments, the antigen binding protein of the present application includes a heavy chain HCDR3, the HCDR3 includes an amino acid sequence as set forth in SEQ ID NO: 3.

In some embodiments, the antigen binding protein of the present application further includes a heavy chain HCDR2, the HCDR2 includes an amino acid sequence as set forth in SEQ ID NO: 77.

In some embodiments, the HCDR2 in the antigen binding protein of the present application includes an amino acid sequence as set forth in any one of SEQ ID NOs: 2 or 26.

In some embodiments, the antigen binding protein of the present application further includes a heavy chain HCDR1, the HCDR1 includes an amino acid sequence as set forth in SEQ ID NO: 1.

In some embodiments, the antigen binding protein of the present application includes heavy chain HCDR1, HCDR2 and HCDR3; where the HCDR1 includes an amino acid sequence as set forth in SEQ ID NO: 1, the HCDR2 includes an amino acid sequence as set forth in SEQ ID NO: 77, and the HCDR3 includes an amino acid sequence as set forth in SEQ ID NO: 3.

In some embodiments, the antigen binding protein of the present application includes heavy chain HCDR1, HCDR2 and HCDR3; where the HCDR1 includes an amino acid sequence as set forth in SEQ ID NO: 1, the HCDR2 includes an amino acid sequence as set forth in any one of SEQ ID NOs: 2 or 26, and the HCDR3 includes an amino acid sequence as set forth in SEQ ID NO: 3.

In some embodiments, the antigen binding protein of the present application includes a heavy chain framework region 1 (H-FR1), the C-terminal of the H-FR1 is connected to the N-terminal of the HCDR1 directly or indirectly, and the H-FR1 includes an amino acid sequence as set forth in any one of SEQ ID NOs: 4 or 17.

In some embodiments, the antigen binding protein of the present application further includes a heavy chain framework region 2 (H-FR2), the H-FR2 is located between the HCDR1 and the HCDR2, and the H-FR2 includes an amino acid sequence as set forth in any one of SEQ ID NOs: 81, 5 or 27.

In some embodiments, the H-FR2 in the antigen binding protein of the present application includes an amino acid sequence as set forth in any one of SEQ ID NOs: 5, 18, 27 or 36.

In some embodiments, the antigen binding protein of the present application further includes a heavy chain framework region 3 (H-FR3), the H-FR3 is located between the HCDR2 and the HCDR3, and the H-FR3 includes an amino acid sequence as set forth in any one of SEQ ID NOs: 6 or 19.

In some embodiments, the antigen binding protein of the present application further includes a heavy chain framework region 4 (H-FR4), the N-terminal of the H-FR4 is connected to the C-terminal of the HCDR3, and the H-FR4 includes an amino acid sequence as set forth in any one of SEQ ID NOs: 7 or 20.

In some embodiments, the antigen binding protein of the present application includes a heavy chain variable region VH, the VH includes an amino acid sequence as set forth in any one of SEQ ID NOs: 73 or 74.

In some embodiments, the antigen binding protein of the present application includes a heavy chain variable region VH, the VH includes an amino acid sequence as set forth in any one of SEQ ID NOs: 8, 21, 28, 37 or 41.

In some embodiments, the antigen binding protein of the present application includes a light chain LCDR3, the LCDR3 includes an amino acid sequence as set forth in SEQ ID NO: 79.

In some embodiments, the LCDR3 in the antigen binding protein of the present application includes an amino acid sequence as set forth in any one of SEQ ID NOs: 11 or 30.

In some embodiments, the antigen binding protein of the present application further includes a light chain LCDR2, the LCDR2 includes an amino acid sequence as set forth in SEQ ID NO: 78.

In some embodiments, the LCDR2 in the antigen binding protein of the present application includes an amino acid sequence as set forth in any one of SEQ ID NOs: 10 or 29.

In some embodiments, the antigen binding protein of the present application further includes a light chain LCDR1, the LCDR1 includes an amino acid sequence as set forth in SEQ ID NO: 9.

In some embodiments, the antigen binding protein of the present application includes light chain LCDR1, LCDR2 and LCDR3; where the LCDR1 includes an amino acid sequence as set forth in SEQ ID NO: 9, the LCDR2 includes an amino acid sequence as set forth in SEQ ID NO: 78, and the LCDR3 includes an amino acid sequence as set forth in SEQ ID NO: 79.

In some embodiments, the antigen binding protein of the present application includes light chain LCDR1, LCDR2 and LCDR3; where the LCDR1 includes an amino acid sequence as set forth in SEQ ID NO: 9, the LCDR2 includes an amino acid sequence as set forth in any one of SEQ ID NOs: 10 or 29, and the LCDR3 includes an amino acid sequence as set forth in any one of SEQ ID NOs: 11 or 30.

In some embodiments, the antigen binding protein of the present application includes a light chain framework region 1 (L-FR1), the C-terminal of the L-FR1 is connected to the N-terminal of the LCDR1 directly or indirectly, and the L-FR1 includes an amino acid sequence as set forth in any one of SEQ ID NOs: 31, 12 or 80.

In some embodiments, the L-FR1 in the antigen binding protein of the present application includes an amino acid sequence as set forth in any one of SEQ ID NOs: 12, 22, 31 or 38.

In some embodiments, the antigen binding protein of the present application further includes a light chain framework region 2 (L-FR2), the L-FR2 is located between the LCDR1 and the LCDR2, and the L-FR2 includes an amino acid sequence as set forth in any one of SEQ ID NOs: 13, 23 or 32.

In some embodiments, the antigen binding protein of the present application further includes a light chain framework region 3 (L-FR3), the L-FR3 is located between the LCDR2 and the LCDR3, and the L-FR3 includes an amino acid sequence as set forth in any one of SEQ ID NOs: 33, 14 or 82.

In some embodiments, the L-FR3 in the antigen binding protein of the present application includes an amino acid sequence as set forth in any one of SEQ ID NOs: 14, 24, 33 or 39.

In some embodiments, the antigen binding protein of the present application further includes a light chain framework region 4 (L-FR4), the N-terminal of the L-FR4 is connected to the C-terminal of the LCDR3, and the L-FR4 includes an amino acid sequence as set forth in any one of SEQ ID NOs: 34 or 15.

In some embodiments, the antigen binding protein of the present application includes a light chain variable region VL, the VL includes an amino acid sequence as set forth in any one of SEQ ID NOs: 75 or 76.

In some embodiments, the antigen binding protein of the present application includes a light chain variable region VL, the VL includes an amino acid sequence as set forth in any one of SEQ ID NOs: 16, 35, 25, or 40.

In some embodiments, the antigen binding protein of the present application includes the HCDR1, HCDR2, HCDR3, LCDR1, LCDR2, and LCDR3, where the HCDR1 includes an amino acid sequence as set forth in SEQ ID NO: 1, the HCDR2 includes an amino acid sequence as set forth in SEQ ID NO: 77, the HCDR3 includes an amino acid sequence as set forth in SEQ ID NO: 3, the LCDR1 includes an amino acid sequence as set forth in SEQ ID NO: 9, the LCDR2 includes an amino acid sequence as set forth in SEQ ID NO: 78, and the LCDR3 includes an amino acid sequence as set forth in SEQ ID NO: 79.

In some embodiments, the antigen binding protein of the present application includes the HCDR1, HCDR2, HCDR3, LCDR1, LCDR2, and LCDR3, where the HCDR1 includes an amino acid sequence as set forth in SEQ ID NO: 1, the HCDR2 includes an amino acid sequence as set forth in any one of SEQ ID NOs: 2 or 26, the HCDR3 includes an amino acid sequence as set forth in SEQ ID NO: 3, the LCDR1 includes an amino acid sequence as set forth in SEQ ID NO: 9, the LCDR2 includes an amino acid sequence as set forth in any one of SEQ ID NOs: 10 or 29, and the LCDR3 includes an amino acid sequence as set forth in any one of SEQ ID NOs: 11 or 30.

In some embodiments, the antigen binding protein of the present application includes a heavy chain variable region VH and a light chain variable region VL, the VH includes an amino acid sequence as set forth in any one of SEQ ID NOs: 73 or 74, and the VL includes an amino acid sequence as set forth in any one of SEQ ID NOs: 75 or 76.

In some embodiments, the antigen binding protein of the present application includes a heavy chain variable region VH and a light chain variable region VL, the VH includes an amino acid sequence as set forth in any one of SEQ ID NOs: 8, 21, 28, 37 or 41, and the VL includes an amino acid sequence as set forth in any one of SEQ ID NOs: 16, 35, 25, or 40.

In some embodiments, the antigen binding protein of the present application includes an antibody or an antigen binding fragment thereof.

In some embodiments, the antigen binding fragment in the antigen binding protein of the present application includes Fab, Fab', F(ab)2, an Fv fragment, F(ab')2, scFv, di-scFv, and/or dAb.

In some embodiments, the antigen binding protein of the present application includes a heavy chain constant region, and the heavy chain constant region is derived from human IgG.

In some embodiments, the antigen binding protein of the present application includes a heavy chain constant region, and the heavy chain constant region is derived from human IgG1.

In some embodiments, the antigen binding protein of the present application includes a heavy chain constant region, where the heavy chain constant region includes an amino acid mutation below: M135Y, S137T and/or T139E, compared to an amino acid sequence as set forth in SEQ ID NO: 42.

In some embodiments, the heavy chain constant region in the antigen binding protein of the present application includes an amino acid sequence as set forth in any one of SEQ ID NOs: 42 or 44.

In some embodiments, the antigen binding protein of the present application includes a heavy chain, and the heavy chain includes an amino acid sequence as set forth in any one of SEQ ID NOs: 45, 47 or 49.

In some embodiments, the antigen binding protein of the present application includes a light chain constant region, and the light chain constant region includes an amino acid sequence as set forth in SEQ ID NO: 43.

In some embodiments, the antigen binding protein of the present application includes a light chain, and the light chain includes an amino acid sequence as set forth in any one of SEQ ID NOs: 46 or 48.

In some embodiments, the antigen binding protein of the present application includes a heavy chain and a light chain, where the heavy chain includes an amino acid sequence as set forth in any one of SEQ ID NOs: 45, 47 or 49, and the light chain includes an amino acid sequence as set forth in any one of SEQ ID NOs: 46 or 48.

In another aspect, the present application further provides a polypeptide comprising the antigen binding protein of the present application.

In another aspect, the present application further provides an immunoconjugate comprising the antigen binding protein of the present application.

In another aspect, the present application further provides an isolated nucleic acid molecule encoding the antigen binding protein of the present application.

In another aspect, the present application further provides a vector comprising the isolated nucleic acid molecule of the present application.

In another aspect, the present application further provides a cell comprising and/or expressing the antigen binding protein of the present application, the polypeptide of the present application, the immunoconjugate of the present application, the isolated nucleic acid molecule of the present application or the vector of the present application.

In another aspect, the present application further provides a method for preparing the antigen binding protein of the present application, which includes culturing the cell of the present application under a condition enabling the expression of the antigen binding protein of the present application. In another aspect, the present application further provides a pharmaceutical composition comprising the antigen binding protein of the present application, the polypeptide of the present application, the immunoconjugate of the present application, the isolated nucleic acid molecule of the present application, the vector of the present application, the cell of the present application, and/or optionally a pharmaceutically acceptable carrier.

In another aspect, the present application further provides use of the antigen binding protein of the present application, the polypeptide of the present application, the immunoconjugate of the present application, the isolated nucleic acid molecule of the present application, the vector of the present application, the cell of the present application and/or the pharmaceutical composition of the present application in the preparation of a drug for preventing, alleviating, and/or treating an IgE-associated disease or disorder.

In another aspect, the present application further provides a method for preventing, alleviating, or treating an IgE-associated disease or disorder, which includes administering to a subject in need thereof the antigen binding protein of the present application, the polypeptide of the present application, the immunoconjugate of the present application, and/or the pharmaceutical composition of the present application.

In another aspect, the present application further provides the antigen binding protein of the present application, the polypeptide of the present application, the isolated nucleic acid molecule of the present application, the vector of the present application, the cell of the present application, the immunoconjugate of the present application and/or the pharmaceutical composition of the present application, which are used for preventing, alleviating or treating an IgE-associated disease or disorder.

In another aspect, the present application further provides a method for detecting or determining IgE, which includes using the antigen binding protein of the present application or the polypeptide of the present application.

In another aspect, the present application further provides a kit for detecting or determining IgE, which includes the antigen binding protein of the present application or the polypeptide of the present application.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows the results of the blocking activity of the antigen binding protein of the present application (a mouse-derived monoclonal antibody) for blocking the binding of IgE to its receptor FcεRIa.
FIG. 2 shows the results of the blocking activity of the antigen binding protein of the present application (a chimeric antibody and its corresponding mouse-derived antibody) for blocking the binding of IgE to its receptor FcεRIa.
FIG. 3 shows the results of the blocking activity of the antigen binding protein of the present application (a humanized antibody, its corresponding chimeric antibody and corresponding mouse-derived antibody) for blocking the binding of IgE to its receptor FcεRIa.
FIG. 4 shows the detection results of the antigen binding protein of the present application (a humanized SE2 antibody) and Omalizumab for blocking the binding of IgE to its receptor FcεRIa.
FIG. 5 shows the detection results of the antigen binding protein of the present application (a humanized SE5 antibody) and Omalizumab for blocking the binding of IgE to its receptor FcεRIa.
FIGs. 6A-6B show the binding affinity of the antigen binding protein of the present application (a humanized SE5ss antibody) to human IgE.
FIGs. 7A-7B show the results of the blocking activity of the antigen binding protein of the present application (a humanized SE5ss antibody) for blocking the binding of IgE to its receptors (FcεRIa and FcεRII).
FIG. 8 shows the detection results of the inhibitory activity of the antigen binding protein of the present application (a humanized SE5ss antibody) for blocking the cell activation mediated by IgE.
FIGs. 9A-9C show the detection results of the inhibitory activity of the antigen binding protein of the present application (a humanized SE5ss antibody) for blocking the cell activation mediated by IgE of different allergens.
FIG. 10 shows the ability of the antigen binding protein of the present application (a humanized SE5ss antibody) to inhibit free human IgE in mice.
FIG. 11 shows the results of the antigen binding protein of the present application (a humanized SE5ss antibody) to alleviate body temperature changes in a mouse model of immediate hypersensitivity.
FIG. 12 shows the ability of the antigen binding protein of the present application (a humanized SE5ss antibody) to inhibit free IgE in a Cynomolgus monkey IgE-inhibiting model.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "antigen binding protein" generally refers to a protein containing an antigen binding portion, and optionally a scaffold or backbone portion that allows the antigen binding portion to adopt a conformation that facilitates the binding of the antigen binding protein to the antigen. The antigen binding protein may include, but not limited to, an antibody, an antigen binding fragment (Fab, Fab', F(ab)2, an Fv fragment, F(ab')2, scFv, di-scFv, and/or dAb), an immunoconjugate, a multispecific antibody (e.g., a bispecific antibody), an antibody fragment, an antibody derivative, an antibody analogue, or a fusion protein, etc., as long as they exhibit the desired antigen binding activity. The "antigen binding protein" of the present application may include a portion that binds the antigen, and optionally a scaffold or framework portion that allows the antigen binding portion to adopt a conformation that facilitates the binding of the antigen binding protein to the antigen.

In the present application, the term "IgE" or "IgE immunoglobulin" or "immunoglobulin E" generally refers to a class of antibodies (or immunoglobulin (Ig)) produced from plasmacytes. A monomer of IgE generally consists of two heavy chains (ε chain) and two light chains, where the ε chain may include four Ig-like constant regions (Cε1-Cε4). In the present application, "IgE" may be an intact IgE or a fragment thereof, and may also be a functional variant, an isoform, a species homolog, a derivative, an analogue of IgE, as well as an analogue having at least one common epitope with IgE. In human, IgE is a polypeptide belonging to the class of antibodies encoded essentially by a publicly known immunoglobulin epsilon gene. IgE may include membrane-anchored (mIgE), or non-membrane-anchored, also known as circulating IgE. In the present application, the term "IgE" may be derived from mammals, and IgE may include human IgE. In the present application, the human IgE has a sequence No. Gene ID: 3497 in the GenBank database.

In the present application, the term "FcεRIα" generally refers to the α chain of a high-affinity receptor of Fc region of immunoglobulin E (IgE) (FcεRI, also known as Fc epsilon RI). FcεRI is a tetramer receptor complex of Fc region capable of binding to the epsilon heavy chain of IgE, which generally consists of one α chain (i.e., FcεRIα), one β chain (FcεRIβ), and two γ chains (FcεRIγ). In general, the α chain may serve as the binding site for an antibody (e.g., IgE), the γ chain may serve as the site for downstream signal initiation, and the β chain may serve to amplify downstream signals. In the present application, "FcεRIα" may be an intact FcεRIα or a fragment thereof, and may also be a functional variant, an isoform, a species homolog, a derivative, an analogue of FcεRIα, as well as an analogue having at least one common epitope with FcεRIα.

In the present application, the term "KD", also known as "*K_{D}*", "K_{D}", "affinity constant" or "equilibrium dissociation constant", generally refers to a value obtained at equilibrium in a titration measurement, or by dividing the dissociation rate constant (k_{d}) by the association rate constant (kₐ). Generally, the association rate constant (kₐ), the dissociation rate constant (k_{d}) and the equilibrium dissociation constant (K_{D}) can be used to represent the binding affinity of the binding protein (e.g., the antigen binding protein of the present application) to the antigen (e.g., human IgE). The methods for determining the binding and dissociation rate constants are well known in the art. For example, the KD value can be determined by Octet, or using other experimental routes and instruments such as BIAcore (Biomolecular Interaction Analysis). In addition, the KD value can be determined by MesoScale Discovery-Solution Equilibrium Titration (MSD-SET), the measurement method being described in Estep P. et, al., MAbs, 2013. 5(2): p. 270-8. In the present application, the KD value can be detected using the KinExA method and software, the measurement method being described in Jonathan K. Fleming et, al., Methods Mol Biol. 2018;1697:1-8.

In the present application, the term "complementarity determining region" or the term "CDR" generally refers to a complementarity determining region in the variable sequence of an antibody. There are three CDRs in each variable region of the heavy chain and the light chain, which are named as CDR1, CDR2 and CDR3 for each variable region. As used herein, a combination of CDRs may refer to a group of three CDRs present in a single variable region that can bind an antigen. The exact boundaries of these CDRs have been defined differently depending on different systems. The system described by Kabat (Kabat et, al., Sequences of Proteins of Immunological Interest National Institutes of Health, Bethesda, Md. (1987) and (1991)) not only provides a definite residue numbering system that can be applied to any variable regions of an antibody, but also provides precise residue boundaries for defining three CDRs. These CDRs may be referred to as Kabat CDRs. Chothia and colleagues (Chothia and Lesk, J.Mol.Biol.196: 901-917 (1987) and Chothia et, al., Nature 342: 877-883 (1989)) found that although there is considerable diversity at the amino acid sequence level, certain sub-portions within Kabat CDRs adopt nearly identical peptide backbone conformations. These sub-portions are named as L1, L2, and L3 or H1, H2, and H3, where "L" and "H" indicate light chain and heavy chain regions, respectively. These regions may be referred to as Chothia CDRs, which have boundaries overlapped with Kabat CDRs. Other boundaries for defining CDRs overlapped with Kabat CDRs have been described by Padlan (FASEB J.9: 133-139 (1995)) and MacCallum (J Mol Biol 262 (5):732-45 (1996)). Other definitions of CDR boundary may not strictly follow one of the above systems, but will still overlap with Kabat CDRs, although they can be shortened or lengthened according to predictions or experimental findings that specific residues or groups of residues or even CDRs as a whole do not significantly affect the antigen binding. CDRs can be defined by KABAT herein, however, it is not excluded that other methods can be used to divide the CDR regions.

In the present application, the term "antibody" generally refers to an immunoglobulin or a fragment or a derivative or variant thereof, encompassing any polypeptides that include an antigen binding site, no matter whether it is produced *in vitro* or *in vivo.* The term includes, but not limited to, polyclonal, monoclonal, mono-specific, multi-specific, non-specific, humanized, single-chain, chimeric, synthetic, recombinant, hybrid, mutated and grafted antibodies. In the present application, the term "antibody" may also include an antibody fragment, such as Fab, F(ab')2, Fv, scFv, Fd, dAb and other antibody fragments that retain the antigen binding function (e.g., specifically binding to human IgE). The antibody of the present application may include antibodies derived from human, mice, monkeys, and/or alpaca. In the present application, the antibody may include its functional variant, isoform, species homolog, derivative, or analogue. For example, the variant in the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to the antibody.

In the present application, the term "antigen binding fragment" generally refers to polypeptide fragments of an immunoglobulin or an antibody that competes with an antigen-bound (i.e., specific binding) intact antibody (i.e., an intact antibody from which they are derived). The antigen binding fragments may include, but not limited to, Fab, Fab', F(ab)2, F(ab')2 and Fv fragments, linear antibodies, single-chain antibodies, diabodies, and multi-specific antibodies formed from antibody fragments.

In the present application, the term "variable region" or "variable domain" generally refers to a portion of the light chain and/or the heavy chain of an antibody, which typically varies significantly among different antibodies and may affect the binding specificity of the antibody to a particular antigen. The variable region may include regions with large sequence variations which are referred to as complementarity determining regions (CDRs) and may also include relatively conserved regions which are usually referred to as framework regions (FRs).

In the present application, the term "connected directly or indirectly" generally refers to relative "direct connection" or "indirect connection". "Direct connection" generally refers to being connected directly. For example, direct connection may be a case in which the substances-to-be-connected (e.g., amino acid sequence segments) are connected directly without spaced components (e.g., amino acid residues or derivatives thereof). For example, an amino acid sequence segment X is directly connected to another amino acid sequence segment Y via the amide bond formed between the amino acid at the C-terminal of the amino acid sequence segment X and the amino acid at the N-terminal of the amino acid sequence segment Y. "Indirect connection" generally refers to a case in which the substances-to-be-connected (e.g., amino acid sequence segments) are connected indirectly with spaced components (e.g., amino acid residues or derivatives thereof). For example, in the antigen binding protein of the present application, the C-terminal of the L-FR1 may be connected to the N-terminal of the LCDR1 directly or indirectly.

In the present application, the term "immunoconjugate" generally refers to other components connected to an antigen binding protein or a fragment thereof. The immunoconjugate may include other polypeptides, therapeutic agents, probes, and/or other antibodies fused or coupled with the antigen binding protein.

In the present application, the term "isolated nucleic acid molecule" generally refers to isolated nucleotides, deoxyribonucleotides or ribonucleotides of any length, or analogues thereof isolated from its natural environment or synthesized artificially.

In the present application, the term "cell" generally refers to an individual cell, cell line, or cell culture that may include or has included a plasmid or vector containing the nucleic acid molecule of the present application, or that can express the antigen binding protein of the present application. The cell may include the progeny of a single cell. Due to natural, accidental, or intentional mutations, the progeny cells may not necessarily be exactly the same as the original parent cells in terms of morphology or genome, as long as they can express the antigen binding protein of the present application. The cells can be obtained by transfecting cells with the vector of the present invention *in vitro.* The cells may be prokaryotic cells (e.g., *E. coli*), or eukaryotic cells (e.g., yeast cells, such as COS cells, Chinese Hamster Ovary (CHO) cells, HeLa cells, HEK293 cells, COS-1 cells, NS0 cells, or myeloma cells).

In the present application, the term "pharmaceutical composition" generally refers to a composition suitable for administering to patients, e.g., human patients. For example, the pharmaceutical composition of the present application may include the antigen binding protein of the present application, the immunoconjugate of the present application, the nucleic acid molecule of the present application, the vector of the present application, and/or the cell of the present application, and optionally a pharmaceutically acceptable carrier. In addition, the pharmaceutical composition may also include one or more (pharmaceutically effective) carriers, stabilizers, excipients, diluents, solubilizers, surfactants, emulsifiers and/or preservatives, and other suitable preparations. The acceptable ingredients of the composition may be non-toxic to the recipient at the dosage and concentration used. The pharmaceutical composition of the present application may include, but not limited to, liquid, frozen and freeze-dried compositions.

In the present application, the term "vector" generally refers to a nucleic acid delivery vehicle into which a polynucleotide encoding a certain protein can be inserted to enable the expression of the protein. The vector can make the genetic elements it carries be expressed in a host cell by transforming, transducing, or transfecting the host cell. For example, the vector may include plasmid; phagemid; Cosmid; artificial chromosomes, such as yeast artificial chromosomes (YACs), bacterial artificial chromosomes (BACs) or P1-derived artificial chromosomes (PACs); phages, such as lambda phages or M13 phages, and animal viruses, and the like. The species of animal viruses used as the vector are retrovirus (including lentivirus), adenovirus, adeno-associated virus, herpes virus (e.g., herpes simplex virus), poxvirus, baculovirus, papilloma virus, papovavirus (e.g., SV40). One vector may contain various elements for controlling the expression, including promoter sequences, transcription initiation sequences, enhancer sequences, selective elements, and reporter genes. In addition, the vector may also contain replication initiation sites. The vector may also probably include ingredients that help its entry into cells, such as virion, lipidosome or protein coat, but not only these substances.

In the present application, the term "pharmaceutically acceptable carrier" generally includes a pharmaceutically acceptable vehicle, excipient, or stabilizer that is non-toxic to the cells or mammals to which they are exposed at the doses and concentrations employed. A physiologically acceptable vector may include suitable substances.

### Detailed Description of the Invention

### Antigen binding protein

In one aspect, the present application provides an antigen binding protein, which may include at least one complementarity determining region (CDR) of a heavy chain variable region (VH) and at least one complementarity determining region (CDR) of a light chain variable region (VL), where the heavy chain variable region includes an amino acid sequence as set forth in any one of SEQ ID NOs: 73 or 74, and the light chain variable region includes an amino acid sequence as set forth in any one of SEQ ID NOs: 75 or 76.

For example, the antigen binding protein of the present application may include one CDR of the heavy chain variable region, where the heavy chain variable region may include an amino acid sequence as set forth in SEQ ID NO: 73 or a variant thereof.

For example, the antigen binding protein of the present application may include two CDRs of the heavy chain variable region, where the heavy chain variable region may include an amino acid sequence as set forth in SEQ ID NO: 73 or a variant thereof.

For example, the antigen binding protein of the present application may include three CDRs of the heavy chain variable region, where the heavy chain variable region may include an amino acid sequence as set forth in SEQ ID NO: 73 or a variant thereof.

For example, the antigen binding protein of the present application may include one CDR of the heavy chain variable region, where the heavy chain variable region may include an amino acid sequence as set forth in SEQ ID NO: 74 or a variant thereof.

For example, the antigen binding protein of the present application may include two CDRs of the heavy chain variable region, where the heavy chain variable region may include an amino acid sequence as set forth in SEQ ID NO: 74 or a variant thereof.

For example, the antigen binding protein of the present application may include three CDRs of the heavy chain variable region, where the heavy chain variable region may include an amino acid sequence as set forth in SEQ ID NO: 74 or a variant thereof.

For example, the CDRs in the heavy chain variable region of the present application may be referred to as HCDRn, where n indicates the numbering of the CDRs, typically may be 1, 2 or 3.

For example, the CDRs in the heavy chain variable region of the present application may include HCDR1, HCDR2, and HCDR3 as described in the present application.

For example, the antigen binding protein of the present application may include a heavy chain HCDR3, where the HCDR3 may include an amino acid sequence as set forth in SEQ ID NO: 3 or a variant thereof.

For example, the antigen binding protein of the present application may include a heavy chain HCDR2, where the HCDR2 may include an amino acid sequence as set forth in SEQ ID NO: 77 or a variant thereof.

For example, the antigen binding protein of the present application may include a heavy chain HCDR2, where the HCDR2 may include an amino acid sequence as set forth in any one of SEQ ID NOs: 2 or 26, or a variant thereof.

For example, the antigen binding protein of the present application may include a heavy chain HCDR1, where the HCDR1 may include an amino acid sequence as set forth in SEQ ID NO: 1 or a variant thereof.

For example, the antigen binding protein of the present application may include heavy chain HCDR3 and HCDR2, where the HCDR3 may include an amino acid sequence as set forth in SEQ ID NO: 3 or a variant thereof, and the HCDR2 may include an amino acid sequence as set forth in SEQ ID NO: 77 or a variant thereof.

For example, the antigen binding protein of the present application may include heavy chain HCDR3 and HCDR2, where the HCDR3 may include an amino acid sequence as set forth in SEQ ID NO: 3 or a variant thereof, and the HCDR2 may include an amino acid sequence as set forth in any one of SEQ ID NOs: 2 or 26, or a variant thereof.

For example, the antigen binding protein of the present application may include heavy chain HCDR2 and HCDR1, where the HCDR2 may include an amino acid sequence as set forth in SEQ ID NO: 77 or a variant thereof, and the HCDR1 may include an amino acid sequence as set forth in SEQ ID NO: 1 or a variant thereof.

For example, the antigen binding protein of the present application may include heavy chain HCDR2 and HCDR1, where the HCDR2 may include an amino acid sequence as set forth in any one of SEQ ID NOs: 2 or 26, or a variant thereof, and the HCDR1 may include an amino acid sequence as set forth in SEQ ID NO: 1 or a variant thereof.

For example, the antigen binding protein of the present application may include heavy chain HCDR1, HCDR2, and HCDR3; where the HCDR1 may include an amino acid sequence as set forth in SEQ ID NO: 1 or a variant thereof, the HCDR2 may include an amino acid sequence as set forth in SEQ ID NO: 77 or a variant thereof, the HCDR3 may include an amino acid sequence as set forth in SEQ ID NO: 3 or a variant thereof.

For example, the antigen binding protein of the present application may include heavy chain HCDR1, HCDR2, and HCDR3; where the HCDR1 may include an amino acid sequence as set forth in SEQ ID NO: 1 or a variant thereof, the HCDR2 may include an amino acid sequence as set forth in any one of SEQ ID NOs: 2 or 26, or a variant thereof, the HCDR3 may include an amino acid sequence as set forth in SEQ ID NO: 3 or a variant thereof.

For example, the antigen binding protein of the present application may include one CDR of the light chain variable region, where the light chain variable region may include an amino acid sequence as set forth in SEQ ID NO: 75 or a variant thereof.

For example, the antigen binding protein of the present application may include two CDRs of the light chain variable region, where the light chain variable region may include an amino acid sequence as set forth in SEQ ID NO: 75 or a variant thereof.

For example, the antigen binding protein of the present application may include three CDRs of the light chain variable region, where the light chain variable region may include an amino acid sequence as set forth in SEQ ID NO: 75 or a variant thereof.

For example, the antigen binding protein of the present application may include one CDR of the light chain variable region, where the light chain variable region may include an amino acid sequence as set forth in SEQ ID NO: 76 or a variant thereof.

For example, the antigen binding protein of the present application may include two CDRs of the light chain variable region, where the light chain variable region may include an amino acid sequence as set forth in SEQ ID NO: 76 or a variant thereof.

For example, the antigen binding protein of the present application may include three CDRs of the light chain variable region, where the light chain variable region may include an amino acid sequence as set forth in SEQ ID NO: 76 or a variant thereof.

For example, the CDRs in the light chain variable region of the present application may be referred to as LCDRn, where n indicates the numbering of the CDRs, typically may be 1, 2 or 3, etc.

For example, the CDRs in the light chain variable region of the present application may include LCDR1, LCDR2 and LCDR3 as described in the present application.

For example, the antigen binding protein of the present application may include a light chain LCDR3, where the LCDR3 may include an amino acid sequence as set forth in SEQ ID NO: 79 or a variant thereof.

For example, the antigen binding protein of the present application may include a light chain LCDR3, where the LCDR3 may include an amino acid sequence as set forth in any one of SEQ ID NOs: 11 or 30, or a variant thereof.

For example, the antigen binding protein of the present application may include a light chain LCDR2, where the LCDR2 may include an amino acid sequence as set forth in SEQ ID NO: 78 or a variant thereof.

For example, the antigen binding protein of the present application may include a light chain LCDR2, where the LCDR2 may include an amino acid sequence as set forth in any one of SEQ ID NOs: 10 or 29, or a variant thereof.

For example, the antigen binding protein of the present application may include a light chain LCDR1, where the LCDR1 may include an amino acid sequence as set forth in SEQ ID NO: 9 or a variant thereof.

For example, the antigen binding protein of the present application includes light chain LCDR3 and LCDR2, where the LCDR3 may include an amino acid sequence as set forth in SEQ ID NO: 79 or a variant thereof, and the LCDR2 may include an amino acid sequence as set forth in SEQ ID NO: 78 or a variant thereof.

For example, the antigen binding protein of the present application includes light chain LCDR3 and LCDR2, where the LCDR3 may include an amino acid sequence as set forth in any one of SEQ ID NOs: 11 or 30, or a variant thereof, and the LCDR2 may include an amino acid sequence as set forth in any one of SEQ ID NOs: 10 or 29, or a variant thereof.

For example, the antigen binding protein of the present application may include light chain LCDR2 and LCDR1, where the LCDR2 may include an amino acid sequence as set forth in SEQ ID NO: 78 or a variant thereof, and the LCDR1 may include an amino acid sequence as set forth in SEQ ID NO: 9 or a variant thereof.

For example, the antigen binding protein of the present application may include light chain LCDR2 and LCDR1, where the LCDR2 may include an amino acid sequence as set forth in any one of SEQ ID NOs: 10 or 29, or a variant thereof, and the LCDR1 may include an amino acid sequence as set forth in SEQ ID NO: 9 or a variant thereof.

For example, the antigen binding protein of the present application may include light chain LCDR1, LCDR2 and LCDR3, where the LCDR1 may include an amino acid sequence as set forth in SEQ ID NO: 9 or a variant thereof, the LCDR2 may include an amino acid sequence as set forth in SEQ ID NO: 78 or a variant thereof, and the LCDR3 may include an amino acid sequence as set forth in SEQ ID NO: 79 or a variant thereof.

For example, the antigen binding protein of the present application may include light chain LCDR1, LCDR2 and LCDR3, where the LCDR1 may include an amino acid sequence as set forth in SEQ ID NO: 9 or a variant thereof, the LCDR2 may include an amino acid sequence as set forth in any one of SEQ ID NOs: 10 or 29, or a variant thereof, and the LCDR3 may include an amino acid sequence as set forth in any one of SEQ ID NOs: 11 or 30, or a variant thereof.

For example, the antigen binding protein of the present application may include HCDR1, HCDR2, HCDR3, LCDR1, LCDR2, and LCDR3, where the HCDR1 may include an amino acid sequence as set forth in SEQ ID NO: 1 or a variant thereof, the HCDR2 may include an amino acid sequence as set forth in SEQ ID NO: 77 or a variant thereof, the HCDR3 may include an amino acid sequence as set forth in SEQ ID NO: 3 or a variant thereof, the LCDR1 may include an amino acid sequence as set forth in SEQ ID NO: 9 or a variant thereof, the LCDR2 may include an amino acid sequence as set forth in SEQ ID NO: 78 or a variant thereof, and the LCDR3 may include an amino acid sequence as set forth in SEQ ID NO: 79 or a variant thereof.

For example, the antigen binding protein of the present application may include HCDR1, HCDR2, HCDR3, LCDR1, LCDR2, and LCDR3, where the HCDR1 may include an amino acid sequence as set forth in SEQ ID NO: 1 or a variant thereof, the HCDR2 may include an amino acid sequence as set forth in any one of SEQ ID NOs: 2 or 26, or a variant thereof, the HCDR3 may include an amino acid sequence as set forth in SEQ ID NO: 3 or a variant thereof, the LCDR1 may include an amino acid sequence as set forth in SEQ ID NO: 9 or a variant thereof, the LCDR2 may include an amino acid sequence as set forth in any one of SEQ ID NOs: 10 or 29, or a variant thereof, and the LCDR3 may include an amino acid sequence as set forth in any one of SEQ ID NOs: 11 or 30, or a variant thereof.

### Framework region (FR)

For example, the light chain variable region of the present application may include framework regions L-FR1, L-FR2, L-FR3, and/or L-FR4.

For example, the light chain variable region of the present application may include framework regions L-FR1, L-FR2, and L-FR3.

For example, the light chain variable region of the present application may include framework regions L-FR1, L-FR2, and L-FR4.

For example, the light chain variable region of the present application may include framework regions L-FR2, L-FR3, and L-FR4.

For example, the light chain variable region of the present application may include framework region L-FR1, L-FR3 and L-FR4.

For example, the light chain variable region of the present application may include framework regions L-FR1, L-FR2, L-FR3, and L-FR4.

For example, the C-terminal of the L-FR1 of the present application may be connected to the N-terminal of the LCDR1 of the present application directly or indirectly. For example, the C-terminal of the L-FR1 of the present application may be connected to the N-terminal of the LCDR1 of the present application directly. For example, the C-terminal of the L-FR1 of the present application may be connected to the N-terminal of the LCDR1 of the present application indirectly. For example, the indirect connection in the present application may include a case in which the amino acid residue at the C-terminal of the L-FR1 of the present application is separated from the amino acid residue at the N-terminal of the LCDR1 of the present application by 1, 2, 3, 4, 5 or more amino acids, and these amino acids may be any naturally occurring or modified amino acids.

For example, the L-FR1 of the present application may include an amino acid sequence as set forth in any one of SEQ ID NOs: 12, 31 or 80, or a variant thereof.

For example, the L-FR1 of the present application may include an amino acid sequence as set forth in any one of SEQ ID NOs: 12, 31, 22, or 38, or a variant thereof.

For example, the L-FR2 of the present application may be located between the LCDR1 of the present application and the LCDR2 of the present application.

For example, the N-terminal of the L-FR2 of the present application may be connected to the C-terminal of the LCDR1 of the present application directly or indirectly, and the C-terminal of the L-FR2 of the present application may be connected to the N-terminal of the LCDR2 of the present application directly or indirectly.

For example, indirect connection may include a case in which the two connected portions (e.g., the L-FR2 of the present application and the LCDR1 of the present application, or the L-FR2 of the present application and the LCDR2 of the present application) are separated by 1, 2, 3, 4, 5 or more amino acids, and these amino acids may be any naturally occurring or modified amino acids.

For example, the L-FR2 of the present application may include an amino acid sequence as set forth in any one of SEQ ID NOs: 13, 32, or 23, or a variant thereof.

For example, the -FR3 of the present application may be located between the LCDR2 of the present application and the LCDR3 of the present application.

For example, the N-terminal of the L-FR3 of the present application may be connected to the C-terminal of the LCDR2 of the present application directly or indirectly, and the C-terminal of the L-FR3 of the present application may be connected to the N-terminal of the LCDR3 of the present application directly or indirectly.

For example, indirect connection may include a case in which the two connected portions (e.g., the L-FR3 of the present application and the LCDR2 of the present application, or the L-FR3 of the present application and the LCDR3 of the present application) are separated by 1, 2, 3, 4, 5 or more amino acids, and these amino acids may be any naturally occurring or modified amino acids.

For example, the L-FR3 of the present application may include an amino acid sequence as set forth in any one of SEQ ID NOs: 14, 33, or 82, or a variant thereof.

For example, the L-FR3 of the present application may include an amino acid sequence as set forth in any one of SEQ ID NOs: 14, 33, 24, or 39, or a variant thereof.

For example, the N-terminal of the L-FR4 of the present application is connected to the C-terminal of the LCDR3 of the present application directly or indirectly.

For example, indirect connection may include a case in which the amino acid residue at the N-terminal of the L-FR4 of the present application is separated from the amino acid residue at the C-terminal of the LCDR3 of the present application by 1, 2, 3, 4, 5 or more amino acids, and these amino acids may be any naturally occurring or modified amino acids.

For example, the L-FR4 of the present application may include an amino acid sequence as set forth in any one of SEQ ID NOs: 15 or 34, or a variant thereof.

For example, the heavy chain variable region of the present application may include framework regions H-FR1, H-FR2, H-FR3, and/or H-FR4.

For example, the light chain variable region of the present application may include framework regions H-FR1, H-FR2, and H-FR3.

For example, the light chain variable region of the present application may include framework regions H-FR1, H-FR2, and H-FR4.

For example, the light chain variable region of the present application may include framework regions H-FR2, H-FR3, and H-FR4.

For example, the light chain variable region of the present application may include framework regions H-FR1, H-FR3, and H-FR4.

For example, the chain variable region of the present application may include framework regions H-FR1, H-FR2, H-FR3, and H-FR4.

For example, the C-terminal of the H-FR1 of the present application may be connected to the N-terminal of the HCDR1 of the present application directly or indirectly. For example, the C-terminal of the H-FR1 of the present application may be connected to the N-terminal of the HCDR1 of the present application directly. For example, the C-terminal of the H-FR1 of the present application may be connected to the N-terminal of the HCDR1 of the present application indirectly. For example, the indirect connection in the present application may include a case in which the amino acid residue at the C-terminal of the H-FR1 of the present application is separated from the amino acid residue at the N-terminal of the HCDR1 of the present application by 1, 2, 3, 4, 5 or more amino acids, and these amino acids may be any naturally occurring or modified amino acids.

For example, the H-FR1 of the present application may include an amino acid sequence as set forth in any one of SEQ ID NOs: 4 or 17, or a variant thereof.

For example, the H-FR2 of the present application may be located between the HCDR1 of the present application and the HCDR2 of the present application.

For example, the N-terminal of the H-FR2 of the present application may be connected to the C-terminal of the HCDR1 of the present application directly or indirectly, and the C-terminal of the H-FR2 of the present application may be connected to the N-terminal of the HCDR2 of the present application directly or indirectly.

For example, the indirect connection in the present application may include a case in which the two connected portions (e.g., the H-FR2 of the present application and the HCDR1 of the present application, or the H-FR2 of the present application and the HCDR2 of the present application) are separated by 1, 2, 3, 4, 5 or more amino acids, and these amino acids may be any naturally occurring or modified amino acids.

For example, the H-FR2 of the present application may include an amino acid sequence as set forth in any one of SEQ ID NOs: 5, 27, or 81, or a variant thereof.

For example, the H-FR2 of the present application may include an amino acid sequence as set forth in any one of SEQ ID NOs: 5, 27, 18, or 36, or a variant thereof.

For example, the H-FR3 of the present application may be located between the HCDR2 of the present application and the HCDR3 of the present application.

For example, the N-terminal of the H-FR3 of the present application may be connected to the C-terminal of the HCDR2 of the present application directly or indirectly, and the C-terminal of the H-FR3 of the present application may be connected to the N-terminal of the HCDR3 of the present application directly or indirectly.

For example, indirect connection may include a case in which the two connected portions (e.g., the H-FR3 of the present application and the HCDR2 of the present application, or the H-FR3 of the present application and the HCDR3 of the present application) are separated by 1, 2, 3, 4, 5 or more amino acids, and these amino acids may be any naturally occurring or modified amino acids.

For example, the H-FR3 of the present application may include an amino acid sequence as set forth in any one of SEQ ID NOs: 6 or 19, or a variant thereof.

For example, the N-terminal of the H-FR4 of the present application may be connected to the C-terminal of the HCDR3 of the present application directly or indirectly.

For example, indirect connection may include a case in which the amino acid residue at the N-terminal of the L-FR4 of the present application is separated from the amino acid residue at the C-terminal of the LCDR3 of the present application by 1, 2, 3, 4, 5 or more amino acids, and these amino acids may be any naturally occurring or modified amino acids.

For example, the H-FR4 of the present application may include an amino acid sequence as set forth in any one of SEQ ID NOs: 7 or 20, or a variant thereof.

For example, when referring to a specific amino acid sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to an amino acid sequence as set forth in any one of SEQ ID NOs: 4-7, 17-20, 27 or 36.

### Variable region, constant region, heavy chain, and light chain

In the present application, the antigen binding protein of the present application may include a heavy chain variable region (VH) and/or a light chain variable region (VL). For example, the heavy chain variable region of the present application may include an amino acid sequence as set forth in any one of SEQ ID NOs: 73 or 74, or a variant thereof. For example, the light chain variable region of the present application may include an amino acid sequence as set forth in any one of SEQ ID NOs: 75 or 76, or a variant thereof.

For example, when referring to a specific amino acid sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to an amino acid sequence as set forth in any one of SEQ ID NOs: 73-76.

For example, the heavy chain variable region of the present application includes an amino acid sequence as set forth in any one of SEQ ID NOs: 73 or 74, or a variant thereof. For example, the light chain variable region of the present application includes an amino acid sequence as set forth in any one of SEQ ID NOs: 75 or 76, or a variant thereof.

For example, the heavy chain variable region of the present application includes an amino acid sequence as set forth in any one of SEQ ID NOs: 8, 21, 28, 37 or 41, or a variant thereof. For example, the light chain variable region of the present application includes an amino acid sequence as set forth in any one of SEQ ID NOs: 16, 35, 25, or 40, or a variant thereof.

For example, the heavy chain variable region of the present application includes a nucleotide sequence as set forth in any one of SEQ ID NOs: 57, 59, 61, 63, or 65, or a variant thereof. For example, the light chain variable region of the present application includes a nucleotide sequence as set forth in any one of SEQ ID NOs: 58, 60, 62, 64, or 66, or a variant thereof.

For example, when referring to a specific amino acid and/or nucleotide sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to an amino acid sequence as set forth in any one of SEQ ID NOs: 8, 16, 21, 25, 28, 35, 37, 40, 41 or 57-66.

For example, the antigen binding protein of the present application may include an antibody heavy chain constant region, and the antibody heavy chain constant region of the present application may be derived from a human IgG heavy chain constant region. For example, the antigen binding protein of the present application may include an antibody heavy chain constant region, and the antibody heavy chain constant region of the present application may be derived from a human IgG1 heavy chain constant region. For example, the antibody heavy chain constant region of the present application may include an amino acid sequence as set forth in SEQ ID NO: 42 or a variant thereof.

For example, when referring to a specific amino acid sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to the amino acid sequence as set forth in SEQ ID NO: 42.

For example, the heavy chain constant region variant of the present application may include an amino acid mutation below: M135Y, S137T, and/or T139E (the numbering of the amino acid sequence is based on the sequence of SEQ ID NO: 42) based on a human IgG1 heavy chain constant region (with its amino acid sequence as set forth in SEQ ID NO: 42).

For example, the antigen binding protein of the present application may include an antibody light chain constant region, and the antibody light chain constant region of the present application may include a human Igκ (Kappa) constant region. For example, the antibody light chain constant region of the present application may include an amino acid sequence as set forth in SEQ ID NO: 43 or a variant thereof.

For example, when referring to a specific amino acid sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to the amino acid sequence as set forth in SEQ ID NO: 43.

For example, the antigen binding protein of the present application may include an antibody heavy chain, and the antibody heavy chain of the present application may include an amino acid sequence as set forth in any one of SEQ ID NOs: 45, 47 or 49, or a variant thereof.

For example, the antigen binding protein of the present application may include an antibody heavy chain, and the antibody heavy chain of the present application may include a nucleotide sequence as set forth in any one of SEQ ID NOs: 67, 69, or 71, or a variant thereof.

For example, when referring to a specific amino acid and/or nucleotide sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to an amino acid sequence as set forth in any one of SEQ ID NOs: 45, 47, 49, 67, 69, or 71.

For example, the antigen binding protein of the present application may include an antibody light chain, and the antibody light chain of the present application may include an amino acid sequence as set forth in any one of SEQ ID NOs: 46 or 48, or a variant thereof.

For example, the antigen binding protein of the present application may include an antibody light chain, and the antibody light chain of the present application may include a nucleotide sequence as set forth in any one of SEQ ID NOs: 68, 70 or 72, or a variant thereof.

For example, when referring to a specific amino acid and/or nucleotide sequence (e.g., a particular sequence), the indicated sequence may also encompass a variant thereof. For example, the variant of the present application may include an amino acid sequence that is at least 80%, at least 85%, at least 90%, at least 95%, at least 96%, at least 97%, at least 98%, at least 98.5%, at least 99%, at least 99.2%, at least 99.4%, at least 99.6%, at least 99.8% or at least 99.9% identical to an amino acid sequence as set forth in any one of SEQ ID NOs: 45-49, or 67-72.

For example, with regard to the antigen binding protein of the present application, the FR1, FR2, FR3, and FR4 may include amino acid sequences selected from any one of the following groups:
1) the H-FR1 includes an amino acid sequence as set forth in SEQ ID NO: 4, the H-FR2 includes an amino acid sequence as set forth in SEQ ID NO: 5, the H-FR3 includes an amino acid sequence as set forth in SEQ ID NO: 6, and the H-FR4 includes an amino acid sequence as set forth in SEQ ID NO: 7; the L-FR1 includes an amino acid sequence as set forth in SEQ ID NO: 12, the L-FR2 includes an amino acid sequence as set forth in SEQ ID NO: 13, the L-FR3 includes an amino acid sequence as set forth in SEQ ID NO: 14, and the L-FR4 includes an amino acid sequence as set forth in SEQ ID NO: 15.
2) the H-FR1 includes an amino acid sequence as set forth in SEQ ID NO: 4, the H-FR2 includes an amino acid sequence as set forth in SEQ ID NO: 27, the H-FR3 includes an amino acid sequence as set forth in SEQ ID NO: 6, and the H-FR4 includes an amino acid sequence as set forth in SEQ ID NO: 7; the L-FR1 includes an amino acid sequence as set forth in SEQ ID NO: 38, the L-FR2 includes an amino acid sequence as set forth in SEQ ID NO: 32, the L-FR3 includes an amino acid sequence as set forth in SEQ ID NO: 33, and the L-FR4 includes an amino acid sequence as set forth in SEQ ID NO: 34.
3) the H-FR1 includes an amino acid sequence as set forth in SEQ ID NO: 17, the H-FR2 includes an amino acid sequence as set forth in SEQ ID NO: 18, the H-FR3 includes an amino acid sequence as set forth in SEQ ID NO: 19, and the H-FR4 includes an amino acid sequence as set forth in SEQ ID NO: 20; the L-FR1 includes an amino acid sequence as set forth in SEQ ID NO: 22, the L-FR2 includes an amino acid sequence as set forth in SEQ ID NO: 23, the L-FR3 includes an amino acid sequence as set forth in SEQ ID NO: 24, and the L-FR4 includes an amino acid sequence as set forth in SEQ ID NO: 15.
4) the H-FR1 includes an amino acid sequence as set forth in SEQ ID NO: 17, the H-FR2 includes an amino acid sequence as set forth in SEQ ID NO: 36, the H-FR3 includes an amino acid sequence as set forth in SEQ ID NO: 19, and the H-FR4 includes an amino acid sequence as set forth in SEQ ID NO: 20; the L-FR1 includes an amino acid sequence as set forth in SEQ ID NO: 31, the L-FR2 includes an amino acid sequence as set forth in SEQ ID NO: 23, the L-FR3 includes an amino acid sequence as set forth in SEQ ID NO: 19, and the L-FR4 includes an amino acid sequence as set forth in SEQ ID NO: 15.
5) the H-FR1 includes an amino acid sequence as set forth in SEQ ID NO: 17, the amino acid sequence as set forth in SEQ ID NO: 36, the H-FR3 includes an amino acid sequence as set forth in SEQ ID NO: 19, and the H-FR4 includes an amino acid sequence as set forth in SEQ ID NO: 20; the L-FR1 includes an amino acid sequence as set forth in SEQ ID NO: 38, the L-FR2 includes an amino acid sequence as set forth in SEQ ID NO: 23, the L-FR3 includes an amino acid sequence as set forth in SEQ ID NO: 39, and the L-FR4 includes an amino acid sequence as set forth in SEQ ID NO: 15.

For example, the antigen binding protein of the present application may include the antibody heavy chain of the present application and the antibody light chain of the present application.

### Properties of antigen binding protein

The antigen binding protein of the present application is capable of binding to IgE (e.g., human IgE) at a KD value of about 2.4×10⁻⁹ M or below. The binding can be determined by the Octet method, MSD-SET method, and/or KinExA method commonly used in the art. For example, the KD value can be determined by MSD-SET, for which the measurement method is described by Estep P. et, al., MAbs, 2013. 5(2): p. 270-8, and the KD value can also be determined by the KinExA method, for which the measurement method is described by Jonathan K. Fleming et, al., Methods Mol Biol. 2018;1697:1-8.

For example, as determined by MSD-SET, the antigen binding protein can bind to a human-derived IgE antigen at a KD value of ≤ 2×10⁻⁹ M, ≤ 1.5 ×10⁻⁹ M, ≤ 1×10⁻⁹ MM, ≤ 8×10⁻¹⁰ M, ≤ 7×10^{- 10} M, ≤ 6×10⁻¹⁰ M, ≤ 5×10⁻¹⁰ M, ≤ 4.5×10⁻¹⁰ M, ≤ 4×10⁻¹⁰ M, ≤ 3.5×10⁻¹⁰ M, ≤ 3×10⁻¹⁰ M, ≤ 2.5×10^{- 10} M, ≤ 2×10⁻¹⁰ M, ≤ 1.8×10⁻¹⁰ M, ≤ 1.5×10⁻¹⁰ M, ≤ 1×10⁻¹⁰ M, < 9×10⁻¹¹ M, < 8×10⁻¹¹ M, ≤ 7×10⁻¹¹ M, ≤ 6×10⁻¹¹ M, ≤ 5×10⁻¹¹ M, ≤ 4×10⁻¹¹ M, ≤ 3×10⁻¹¹ M, ≤ 2×10⁻¹¹ M, ≤ 1×10⁻¹¹ M, ≤ 9×10⁻¹² M.

For example, as determined by the KinExA method, the antigen binding protein of the present application can bind to the IgE at a KD value of, e.g., ≤ 3.5×10⁻¹² M, ≤ 3.4×10⁻¹² M, ≤ 3.3×10⁻¹² M, ≤ 3.2×10⁻¹² M, ≤ 3.1×10⁻¹² M, ≤ 3×10⁻¹² M, ≤ 2.9×10⁻¹² M, ≤ 2.8×10⁻¹² M, ≤ 2.7×10⁻¹² M, ≤ 2.6×10^{- 12} M, ≤ 2.5×10⁻¹² M, ≤ 2.410⁻¹² M, ≤ 2.3×10⁻¹² M, ≤ 2.2×10⁻¹² M, ≤ 2.1×10⁻¹² M, ≤ 2.0×10⁻¹² M, ≤ 1.9×10⁻¹² M, ≤ 1.8×10⁻¹² M, ≤ 1.7×10⁻¹² M, ≤ 1.6×10⁻¹² M, ≤ 1.5×10⁻¹² M, ≤ 1.4×10⁻¹² M, ≤ 1.3×10^{- 12} M, ≤ 1.2×10⁻¹² M or ≤ 1.1×10⁻¹² M.

The antigen binding protein of the present application is capable of inhibiting the binding of IgE to its receptors FcεRIa and/or FcεRII.

For example, as detected by the ELISA method, the antigen binding protein of the present application can inhibit the binding of IgE to its receptor FcεRIa at an IC₅₀ value of ≤ 2000 ng/mL, ≤ 1800 ng/mL, ≤ 1500 ng/mL, ≤ 1300 ng/mL, ≤ 1000 ng/mL, ≤ 950 ng/mL, ≤ 900 ng/mL, ≤ 850 ng/mL, ≤ 800 ng/mL, ≤ 750 ng/mL, ≤ 700 ng/mL, ≤ 650 ng/mL, ≤ 600 ng/mL, ≤ 550 ng/mL, ≤ 500 ng/mL, ≤ 450 ng/mL, ≤ 430 ng/mL, ≤ 400 ng/mL.

For example, as detected by the ELISA method, the antigen binding protein of the present application can inhibit the binding of IgE to its receptor FcεRIa at an IC₅₀ value of ≤ 4.9_{×}10⁻⁹ M, ≤ 4.5 ×10⁻⁹ M, ≤ 4.0×10⁻⁹ M, ≤ 3.5×10⁻⁹ M, ≤ 3.0×10⁻⁹ M, ≤ 2.5 ×10⁻⁹ M, ≤ 2.0×10⁻⁹ M, ≤ 1.5 ×10⁻⁹ M, ≤ 1.0×10⁻⁹ M, ≤ 9.0×10⁻¹⁰ M, ≤ 8.0×10⁻¹⁰ M, ≤ 7.0×10⁻¹⁰ M, ≤ 6.0×10⁻¹⁰ M, ≤ 5.0×10⁻¹⁰ M, ≤ 4.0×10⁻¹⁰ M, ≤ 3.0×10⁻¹⁰ M, ≤ 2.5×10⁻¹⁰ M.

For example, as detected by the ELISA method, the antigen binding protein of the present application can inhibit the binding of IgE to its receptor FcεRIa at an IC₅₀ value of, e.g., ≤ 35 ng/mL, ≤ 34 ng/mL, ≤ 33 ng/mL, ≤ 32.5 ng/mL, ≤ 32.5 ng/mL, ≤ 32.4 ng/mL, ≤ 32.3 ng/mL, ≤ 32.2 ng/mL, ≤ 32.1 ng/mL, ≤ 32.0 ng/mL, ≤ 31 ng/mL, ≤ 30 ng/mL, ≤ 29 ng/mL, ≤ 28 ng/mL, ≤ 27 ng/mL, ≤ 26 ng/mL, ≤ 25 ng/mL, ≤ 24 ng/mL, ≤ 23 ng/mL, ≤ 22 ng/mL, ≤ 21 ng/mL, ≤ 20 ng/mL, ≤ 19 ng/mL, ≤ 18 ng/mL, ≤ 17 ng/mL, ≤ 16 ng/mL, ≤ 15 ng/mL, ≤ 14 ng/mL, ≤ 13 ng/mL, ≤ 12 ng/mL, ≤ 11 ng/mL or ≤ 10 ng/mL.

For example, as detected by the ELISA method, the antigen binding protein of the present application can inhibit the binding of IgE to its receptor FcεRII at an IC₅₀ value of, e.g., <_ 4.0 µg/mL, ≤ 3.9 µg/mL, ≤ 3.8 µg/mL, ≤ 3.7 µg/mL, ≤ 3.6 µg/mL, ≤ 3.5 µg/mL, ≤ 3.4 µg/mL, ≤ 3.3 µg/mL, ≤ 3.2 µg/mL, ≤ 3.1 µg/mL or ≤ 3.0 µg/mL.

For example, the antigen binding protein of the present application may include an antibody or an antigen binding fragment thereof. For example, the antibody of the present application may include, but not limited to, a recombinant antibody, a monoclonal antibody, a human antibody, a mouse-derived antibody, a humanized antibody, a chimeric antibody, a camelid single domain antibody, a bispecific antibody, a single-chain antibody, a diabody, a triabody or a tetrabody.

For example, the antibody of the present application may be a humanized antibody. For example, the antigen binding protein of the present application may be an antibody or a variant, a derivative, an analogue or a fragment thereof that immuno-specifically binds to an antigen of interest (e.g., a human IgE antigen) and includes a framework region (FR) substantially having an amino acid sequence of a human antibody and a complementarity determining region (CDR) substantially having an amino acid sequence of a non-human antibody. "Substantially" used herein, in the case of a CDR, refers to that the amino acid sequence of the CDR is at least 80%, at least 85%, at least 90%, at least 95%, at least 96%, at least 97%, at least 98%, at least 98.5% or at least 99% identical to the amino acid sequence of the CDR of a non-human antibody. The humanized antibody of the present application may substantially include all, at least one and usually two variable domains (Fab, Fab', F(ab')2, Fv), where all or substantially all the CDR regions correspond to CDR regions of non-human immunoglobulin (i.e., antibodies) and all or substantially all the framework regions are framework regions having consensus sequences of human immunoglobulin. For example, the humanized antibody may also include at least a part of immunoglobulin constant regions (e.g., Fc), which are generally the constant regions of human immunoglobulin. In some embodiments, the humanized antibody contains at least variable domains of a light chain or/and a heavy chain. The antibody may also include CH1, hinge, CH2, CH3 and CH4 regions of a heavy chain. For example, the humanized antibody may contain only a humanized light chain. For example, the humanized antibody may contain only a humanized heavy chain. For example, the humanized antibody may contain only a humanized variable domain of a light chain and/or a humanized heavy chain.

For example, the antigen binding fragment of the present application may include Fab, Fab', F(ab)2, an Fv fragment, F(ab')2, scFv, di-scFv, and/or dAb.

### Polypeptide and immunoconjugate

In another aspect, the present application provides a polypeptide, which may include the antigen binding protein of the present application. The polypeptide of the present application may also include other peptide fragments or protein fragments. For example, the polypeptide may also include other scaffold or backbone portions that facilitate the binding conformation of the antigen binding protein to the antigen. In some cases, the polypeptide of the present application may also include portions binding to other antigens, and/or protein fragments having other functions. For example, the polypeptide of the present application may be fusion protein, multi-specific antibodies, and/or other recombinant protein.

In another aspect, the present application provides an immunoconjugate, which may include the antigen binding protein of the present application or the polypeptide of the present application. For example, the immunoconjugate of the present application may include other polypeptides, therapeutic agents (e.g., cytotoxic molecules), probes and/or other antibodies fused or coupled with the antigen binding protein. For example, the immunoconjugate of the present application may include pharmaceutically acceptable markers, detection reagents, and/or therapeutic agents.

### Nucleic acid, vectors, host cells and preparation method

In another aspect, the present application provides one or more isolated nucleic acid molecules, which may encode the antigen binding protein or polypeptide of the present application. For example, each nucleic acid molecule in the one or more nucleic acid molecules may encode an intact isolated antigen binding protein or polypeptide and may also encode a part thereof. The nucleic acid molecules of the present application may be isolated. In the present application, a variety of methods known in the art can be used to prepare the nucleic acid encoding the antigen binding protein or the polypeptide.

In another aspect, the present application provides one or more vectors, which include the one or more nucleic acid molecules of the present application. Each vector may include one or more of the nucleic acid molecules. In addition, the vectors may also include other genes, e.g., marker genes allowing the selection of the vector in appropriate host cells and under appropriate conditions. In addition, the vectors may contain various elements for controlling the expression, including promoter sequences, transcription initiation sequences, enhancer sequences, selection elements, and reporter genes. In addition, the vectors may also contain replication initiation sites. Moreover, the vectors may include, e.g., plasmid, cosmid, virus, phage, or other vectors commonly used in, e.g., genetic engineering.

In another aspect, the present application provides a cell, which may include the one or more nucleic acid molecules of the present application and/or the one or more vectors of the present application. For example, each kind of or each of the cells may include one or one kind of the nucleic acid molecules or vectors of the present application. For example, each kind of or each of the cells may include multiple (e.g., two or more) or multiple kinds of (e.g., two or more kinds of) the nucleic acid molecules or vectors of the present application. For example, the vectors of the present application can be introduced into the cells, e.g., prokaryotic cells (e.g., bacterial cells), CHO cells, NS/0 cells, HEK293 cells, or other eukaryotic cells, such as cells from plants, fungi or yeast cells, and the like. The vectors of the present application can be introduced into the cells by methods known in the art, such as electroporation, lipofectine transfection, lipofectamin transfection, and the like.

For example, the vectors may include plasmid; phagemid; Cosmid; artificial chromosomes, such as yeast artificial chromosomes (YACs), bacterial artificial chromosomes (BACs) or P1-derived artificial chromosomes (PACs); phages, such as lambda phages or M13 phages, and animal viruses, and the like. The species of animal viruses used as the vectors are retrovirus (including lentivirus), adenovirus, adeno-associated virus, herpes virus (e.g., herpes simplex virus), poxvirus, baculovirus, papilloma virus, papovavirus (e.g., SV40). Further for example, the vector may contain various elements for controlling the expression, including promoter sequences, transcription initiation sequences, enhancer sequences, selection elements, and reporter genes. In addition, the vectors may also contain replication initiation sites. Moreover, the vectors may also include ingredients that help its entry into cells, such as virion, lipidosome or protein coat, but not only these substances.

For example, the cells may include prokaryotic cells, yeast cells or higher eukaryotic cells. Prokaryotes suitable for this purpose include Gram-negative bacteria and Gram-positive bacteria, e.g., enterobacteria, such as *E. coli*, *Enterobacter*, *Erwinia*, *Klebsiella*, *Proteus*, *Salmonella*, *Serratia marcescens*, and *Shigella*, as well as *Bacillus*, *Pseudomonas*, and *Streptomyces.*

For example, the cells may include mammalian host cell lines. For example, monkey kidney cells, human embryonic kidney cell lines, baby hamster kidney cells, Chinese hamster ovary cells, mouse sertoli cells, Hela cells (HELA), dog kidney cells, human lung cells, human liver cells, mouse breast cancer cells or NSO cells.

For example, the cells may also include cells transfected with the vector of the present application *in vitro.* For example, the cells may be bacterial cells (e.g., *E. coli*)*,* yeast cells or other eukaryotic cells, e.g., COS cells, Chinese hamster ovary (CHO) cells, CHO-K1 cells, LNCAP cells, HeLa cells, HEK293 cells, COS-1 cells, NS0 cells or myeloma cells. In some embodiments, the cells may be mammalian cells. For example, the mammalian cells may be HEK293 cells.

### Pharmaceutical composition, and application

In another aspect, the present application further provides a method for preparing the antigen binding protein of the present application, which may include culturing the cell of the present application under a condition enabling the expression of the antigen binding protein of the present application.

In another aspect, the present application further provides use of the antigen binding protein of the present application, the nucleic acid molecule of the present application, the vector of the present application, the cell of the present application and/or the pharmaceutical composition of the present application in the preparation of a drug for preventing, alleviating and/or treating an IgE-associated disease or disorder.

In another aspect, the present application further provides a method for preventing, alleviating or treating an IgE-associated disease or disorder, which may include administering to a subject in need thereof the antigen binding protein of the present application, the nucleic acid molecule of the present application, the vector of the present application, the cell of the present application and/or the pharmaceutical composition of the present application.

In another aspect, the present application further provides the antigen binding protein of the present application, the nucleic acid molecule of the present application, the vector of the present application, the cell of the present application and/or the pharmaceutical composition of the present application, which may for use in preventing, alleviating or treating an IgE-associated disease or disorder.

For example, the IgE-associated disease or disorder may include diseases or disorders for which Omalizumab (Trade Name: Xolair) is approved for treatment worldwide (e.g., the United States, China, Europe, etc.). For example, the diseases or disorders may include allergic diseases.

For example, the subject may include human and non-human animals. For example, the subject may include, but not limited to, cat, dog, horse, pig, cow, sheep, rabbit, mouse, rat, or monkey.

In another aspect, the present application further provides a pharmaceutical composition, which includes the antigen binding protein of the present application, the nucleic acid molecule of the present application, the vector of the present application, and/or the cell of the present application, as well as optionally a pharmaceutically acceptable carrier.

For example, the pharmaceutical composition may additionally contain one or more other therapeutic agents suitable for treating or preventing IgE-associated diseases or disorders.

For example, the pharmaceutically acceptable carrier reinforces or stabilizes the composition, or facilitates the preparation of the composition. For example, the pharmaceutically acceptable carrier may include physiologically compatible solvents, dispersion media, coating materials, antibacterial agents and antifungal agents, isotonic agents, and absorption retarders, etc.

For example, the administration can be done in different ways, such as intravenously, intraperitoneally, subcutaneously, intramuscularly, locally, or intradermally.

For example, the pharmaceutical composition of the present application can be administered by a variety of methods known in the art, depending on the desired results, the administration route and/or the administration mode. For example, the administration may be intravenous, intramuscular, intraperitoneal, or subcutaneous administration, or administration near the target sites. For example, the pharmaceutical composition is formulated so that it can be administered into eyes intravitreally. For example, depending on the administration route, the antigen binding protein (e.g., antibodies, bispecific and multi-specific molecules) can be coated with materials to protect the compounds from the action of acids or other natural conditions which may inactivate the compounds.

For example, the pharmaceutical composition of the present application may be sterile and fluid. For example, proper flowability can be maintained by using coating materials such as lecithin, or in the case of dispersions, by maintaining the required particle size, and by using surfactants.

For example, isotonic agents, e.g., saccharides and polyalcohols, such as mannitol, sorbitol or sodium chloride, can be included in the composition. For example, the prolonged absorption of the injectable pharmaceutical composition can be achieved by including in the pharmaceutical composition a reagent which delays the absorption (e.g., aluminum monostearate or gelatin).

For example, the antigen binding protein of the present application, the nucleic acid molecule of the present application, the vector of the present application and/or the cell of the present application are mixed with optionally pharmaceutically acceptable vehicles, excipients or stabilizers to prepare the composition for storage. For example, the pharmaceutical composition of the present application may be in a form of a freeze-dried preparation or aqueous solution.

For example, the pharmaceutically acceptable carrier of the present application may include a pharmaceutically acceptable vehicle, excipient or stabilizer.

For example, the acceptable vehicle, excipient or stabilizer of the present application is non-toxic to the recipient at the dosage and concentration used, and includes buffering agents such as phosphates, citrates, acetates, and other organic acids.

For example, the pharmaceutical composition containing the antigen binding protein of the present application may be in a water-soluble form.

For example, the pharmaceutical composition for *in vivo* administration may be sterile. This can be easily achieved by filtration over a sterile filter membrane or other methods. For example, the administration of the pharmaceutical composition containing the antigen binding protein of the present application in a form of sterile aqueous solution can done in a variety of ways, including but not limited to, orally, subcutaneously, intravenously, intranasally, intraaurally, transdermally, locally (e.g., gel, ointment, lotion, cream, etc.), intraperitoneally, intramuscularly, intrapulmonarily, parenterally, rectally, or intraocularly. In some cases, for treating wounds and inflammations for example, the antigen binding protein of the present application can be applied directly as a solution or spray.

For example, the pharmaceutical composition of the present application can be prepared by conventional practical methods well known in the art. For example, the pharmaceutical composition of the present application can be prepared under GMP (Good Manufacturing Practices) conditions. Generally, a therapeutically effective dose or an effective dose of IgE binding protein is used in the pharmaceutical composition of the present application. For example, the IgE binding protein can be formulated into a pharmaceutically acceptable dosage form by conventional methods known to those skilled in the art. The dosage regimen is adjusted to provide optimal desired responses (e.g., therapeutic responses). The actual dose level of the active ingredient in the pharmaceutical composition of the present application can vary to obtain an amount that can effectively realize the desired therapeutic responses, the composition, and the mode of administration for particular subjects, but is not toxic to the patients. The dose level selected depends on a variety of pharmacokinetic factors, e.g., the activity of the particular composition or esters, salts, or amides thereof used in the present application, the route of administration, the time of administration, the rate of excretion of the particular compound being used, the duration of treatment, other drugs, compounds and/or substances used in combination with the particular composition being used, the age, gender, weight, condition, general health and previous medical history of the subject being treated, as well as other factors.

For example, subcutaneous administration can be used in situations where the patient can administer the pharmaceutical composition on his/her own. Many protein therapeutic agents are not sufficiently effective to allow formulation of a therapeutically effective dose for subcutaneous administration of the maximum acceptable volume. The antigen binding protein disclosed in the present application can be suitable for subcutaneous administration, for example, to increase the titer, improve the half life of plasma, and increase the solubility.

As known in the art, the protein therapeutic agents can be delivered by IV infusion or bolus injection. The antigen binding protein disclosed in the present application can also be delivered by such methods.

In another aspect, the present application further provides a method for detecting IgE in a sample, which includes administering the antigen binding protein of the present application.

For example, the sample obtained from the subject is contacted with the antigen binding protein of the present application (e.g., IgE binding protein). For example, the IgE binding protein is labelled with a detectable marker or a reporter molecule marker, or an anti-IgE binding protein is used as a capturing ligand to selectively separate IgE from the sample of a patient. Alternatively, unlabelled anti-IgE binding protein can be used in detection applications by binding to a secondary antibody, which itself has a detectable marker. The detectable marker or reporter molecule may be a radioisotope, such as ³H, ¹⁴C, ³²P, ³⁵S or ¹²⁵I; or may be a fluorescent or chemiluminescent component, such as fluorescein isothiocyanate, rhodamine; or an enzyme, such as alkaline phosphatase, β-galactosidase, horseradish peroxidase or luciferase. Specific exemplary assays that can be used to detect or measure IgE in a sample include enzyme-linked immunosorbent assay (ELISA), radioimmunoassay (RIA), and fluorescence-activated cell sorting (FACS).

Samples that may be used in the IgE assay of the present application may include any tissues or fluid samples obtained from a subject in a normal or pathological conditions, which contain detectable amounts of IgE protein or fragments thereof. For example, the level of the IgE protein in a particular sample obtained from a healthy subject (e.g., a subject without an IgE-related disease) may be measured to initially establish the baseline or standard level of IgE. This baseline level of IgE is then compared with respect to the level of IgE measured in a sample obtained from an individual that is suspected of having an IgE-related disease or disorder or symptoms associated with that disorder. The IgE binding protein may contain no additional markers, or may contain N-terminal or C-terminal markers. For example, the markers are biotin. In a binding assay, the position of the marker (if present) can be used to determine the orientation of the peptide relative to the surface on which the peptide is bound. For example, if a surface is coated with avidin, the peptide containing N-terminal biotin will keep the C-terminal portion of the peptide away from the surface.

Without intending to be limited by any theory, the following examples are only to illustrate various technical schemes of the present application, and not intended to limit the scope of the present application.

### Examples

### Example 1 Preparation of the antigen binding protein of the present application

### 1-1 Preparation of anti-human IgE mouse-derived monoclonal antibodies

### (1) Immunization of mice

Four-week-old BALB/c mice were subcutaneously injected with 20 µg of recombinantly expressed Fc region (CH2-4) protein of human IgE (IgE-Fc, SEQ ID NO: 42) (in Freund's complete adjuvant) once every three to four weeks for a total of five injections. Finally, a single dose of 20 µg of recombinant human IgE-Fc protein was injected intraperitoneally.

### (2) Determination of the binding of mouse serum to human IgE-Fc antigen by ELISA method

ELISA plates (Costar) were coated with recombinant human IgE-Fc protein at room temperature overnight. The coating solution was discarded. The wells were blocked with 2.5% skimmed milk dissolved in phosphate buffered saline (PBS) for 0.5 hours, and washed with PBS containing 0.05% Tween-20. Then, diluted sera and irrelevant antibodies (Irrevalent mAb) as the negative control were added separately and incubated at room temperature for 1 hour. The wells were washed with PBS containing 0.05% Tween-20. 50 µl of HRP-labelled goat-anti-mouse IgG polyclonal antibodies (Jackson Laboratory) were then added to each well as the antibodies for detection. After serum testing, a mouse containing a high level of anti-recombinant human IgE-Fc protein antibody sera was identified.

### (3) Cell fusion, screening, and preparation of mouse-derived monoclonal antibodies

After identifying a mouse containing a high level of anti-human IgE-Fc antibody sera through serum testing, the spleen was taken from the mouse and fused with a mouse myeloma Sp2/0 cell line. 5×10⁸ of Sp2/0 cells and 5×10⁸ of spleen cells were mixed and fused in a solution containing 50% of polyethylene glycol (PEG, with a molecular weight of 1450) and 5% of dimethyl sulfoxide (DMSO). The number of spleen cells was adjusted with Iscove medium (containing 10% fetal calf serum, 100 units/mL penicillin, 100 µg/mL streptomycin, 10 mM hypoxanthine, 0.4 µM aminopterin and 1.6 mM thymidine) to 7.5×10⁵/mL, and added into the wells of a 96-well culture plate at 0.2 ml. The culture plate was placed in an incubator at 37°C and 5% CO₂. After 10 days, the media were taken out of the wells and tested for the binding ability of the mouse-derived antibodies to human IgE-Fc, so as to screen out the positive wells bound to human IgE-Fc. The fusion cells in the wells containing the antibodies that can bind to human IgE-Fc were then subcloned, and screened likewise by the ELISA method to obtain hybridoma cell lines expressing high-affinity mouse monoclonal antibodies.

Purified mouse monoclonal antibodies were prepared to obtain antibody samples to be tested. Cell clones that produce specific antibodies were cultured in a RPMI1640 medium supplemented with 10% FCS. When the cell density reached about 5×10⁵ cells/ml, the medium was replaced with a serum-free medium. After 2 to 4 days, the cultured medium was centrifuged to collect the culture supernatant. The antibodies were purified by using a protein G column. The eluent of the monoclonal antibodies was dialyzed with 150 mM of NaCl. The dialyzed solution was filtered over a 0.2 µm filter for sterilization, to obtain the antibody samples to be tested. Monoclonal antibodies SE3(11/5), SE5(4/23), SE2, and SE5 were prepared.

### 1-2 Preparation of anti-human IgE chimeric antibody

### (1) Cloning of a heavy chain variable region of a mouse-derived antibody

To obtain DNA sequences encoding the heavy chain and light chain variable regions of a mouse-derived antibody, mRNA was isolated from mouse hybridoma cells by using an mRNA purification kit (NEB) to prepare cDNA (SMARTer RACE kit, Clontech). Heavy chain variable region DNA fragments were cloned from cDNA through polymerase chain reaction (PCR). The 5'-primer for PCR was a mixed solution consisting of 0.4 µM of primer 1 (5'-ctaatacgactcactatagggcAAGCAGTGGTATCAACGCAGAGT-3', as set forth in SEQ ID NO: 53) and 2 µM of primer 2 (5'-ctaatacgactcactatagggc-3', as set forth in SEQ ID NO: 54). The 3'-primer for PCR was primer 3 (5' -catcccagggtcaccatggagtta-3', as set forth in SEQ ID NO: 55). The primer 3 was homologous but antisense to the mouse IgG1 heavy chain constant region. The heavy chain variable region DNA fragments obtained from gel purification were cloned into TOPO-TA vectors (Invitrogen) and sequenced to obtain the amino acid sequence (as set forth in SEQ ID NO: 8) and nucleotide sequence (as set forth in SEQ ID NO: 57) of the variable region encoding the heavy chain of the SE2 mouse hybridoma, the amino acid sequences of its complementarity determining region, HCDR1 (as set forth in SEQ ID NO: 1), HCDR2 (as set forth in SEQ ID NO: 2) and HCDR3 (as set forth in SEQ ID NO: 3), as determined by means of Kabat definition, and the amino acid sequence (as set forth in SEQ ID NO: 28) and nucleotide sequence (as set forth in SEQ ID NO: 61) of the variable region encoding the heavy chain of the SE5 mouse hybridoma, and the amino acid sequences of its complementarity determining region, HCDR1 (as set forth in SEQ ID NO: 1), HCDR2 (as set forth in SEQ ID NO: 26) and HCDR3 (as set forth in SEQ ID NO: 3). For the definition of the complementarity determining region, see Kabat E. et al., Sequences of Proteins of Immunological Interest, Ed. 5, U.S. Department of Health and Human Services, NIH Publication No. 91-3242.

### (2) Cloning of a light chain variable region of a mouse-derived antibody

By using a method similar to that in step (1), light chain variable region DNA fragments were cloned from cDNA by using the above 5'-primer mixed solution (primer 1 and primer 2) and another 3'-primer that is homologous but antisense to the mouse immunoglobulin light chain constant region (primer 4, 5'-gactgaggcacctccagatgttaa-3', as set forth in SEQ ID NO: 56). These obtained DNA fragments were cloned into TOPO-TA vectors and sequenced to obtain the amino acid sequence (as set forth in SEQ ID NO: 16) and nucleotide sequence (as set forth in SEQ ID NO: 58) of the variable region encoding the light chain of the SE2 mouse hybridoma, and the amino acid sequences of its complementarity determining region, LCDR1 (as set forth in SEQ ID NO: 9), LCDR2 (as set forth in SEQ ID NO: 10) and LCDR3 (as set forth in SEQ ID NO: 11), and the amino acid sequence (as set forth in SEQ ID NO: 35) and nucleotide sequence (as set forth in SEQ ID NO: 62) of the variable region encoding the light chain of the SE5 mouse hybridoma, and the amino acid sequences of its complementarity determining region, LCDR1 (as set forth in SEQ ID NO: 9), LCDR2 (as set forth in SEQ ID NO: 29) and LCDR3 (as set forth in SEQ ID NO: 30).

### (3) Preparation of chimeric antibodies

The sequences of the heavy chain and light chain variable regions of the mouse-derived monoclonal antibodies obtained from the cloning in step 2) were inserted into pCDNA3.1 (purchased from MiaoLingBio) eukaryotic expression vectors containing a signal peptide (as set forth in SEQ ID NO: 50) and a human IgG1 heavy chain constant region (as set forth in SEQ ID NO: 42), or containing a signal peptide (as set forth in SEQ ID NO: 50) and a human Kappa light chain constant region (as set forth in SEQ ID NO: 43) by a seamless cloning method, respectively. After being confirmed as correct by DNA sequencing, plasmids were prepared using NucleoBond Xtra Midi Plus (MACHEREY-NAGEL) plasmid midi preparation kit after amplification in DH5α strain. The prepared chimeric monoclonal antibody heavy chain and light chain plasmids were co-transfected into Expi293 cells (purchased from Thermo fisher) using PEI (PolyScience) to transiently express the monoclonal antibodies. The transiently expressed antibodies were purified by using a protein A column, and the eluent of the monoclonal antibodies was dialyzed with PBS. The dialyzed solution was filtered over a 0.2 µm filter for sterilization, to obtain the antibody samples to be tested.

### 1-3 Preparation of anti-human IgE humanized antibodies

### (1) Humanization of anti-human IgE mouse-derived monoclonal antibodies

Based on the homology modeling of the antibody variable region, key amino acid sites that could potentially affect antigen-antibody interactions were identified, and reverse mutation was subsequently performed on these key amino acid sites. Humanized amino acid sequences of the variable regions of the SE2 and SE5 antibodies were obtained by using the CDR transplantation method, where the amino acid sequence of the humanized SE2 heavy chain variable region was as set forth in SEQ ID NO: 21, the amino acid sequence of the humanized SE2 light chain variable region was as set forth in SEQ ID NO: 25, the amino acid sequence of the humanized SE5 heavy chain variable region was as set forth in SEQ ID NO: 37, the amino acid sequence of the humanized SE5 light chain variable region was as set forth in SEQ ID NO: 40. The amino acid sequences were translated inversely and the obtained DNA sequences were synthesized, the humanized nucleotide sequence of the SE2 heavy chain variable region was as set forth in SEQ ID NO: 21, the humanized nucleotide sequence of the SE2 light chain variable region was as set forth in SEQ ID NO: 60, the humanized nucleotide sequence of the SE5 heavy chain variable region was as set forth in SEQ ID NO: 63, and the humanized nucleotide sequence of the SE5 light chain variable region was as set forth in SEQ ID NO: 64, and the obtained DNA was used for the construction of recombinant antibody expression vectors.

### (2) Preparation of anti-human IgE humanized antibodies

The heavy chain and light chain variable regions of the humanized monoclonal antibodies obtained from step 1) were inserted into pCDNA3.1 (purchased from MiaoLingBio) eukaryotic expression vectors containing a signal peptide (as set forth in SEQ ID NO: 50) and a human IgG1 heavy chain constant region (as set forth in SEQ ID NO: 42), or containing a signal peptide (as set forth in SEQ ID NO: 50) and a human Kappa light chain constant region (as set forth in SEQ ID NO: 43) by a seamless cloning method, respectively. After being confirmed as correct by DNA sequencing, plasmids were prepared using NucleoBond Xtra Midi Plus (MACHEREY-NAGEL) plasmid midi preparation kit. The prepared humanized monoclonal antibody heavy chain and light chain plasmids were co-transfected into Expi293 cells (purchased from Thermo fisher) using PEI (PolyScience) to transiently express the monoclonal antibodies. The transiently expressed antibodies were purified by using a protein A column, and the eluent of the monoclonal antibodies was dialyzed with PBS. The dialyzed solution was filtered over a 0.2 µm filter for sterilization, to obtain the antibody samples to be tested.

### (3) Modified humanized antibodies

It was found through sequence analysis of the antibody variable region that, N53 in the CDR2 region of the SE5 heavy chain (as set forth in SEQ ID NO: 26) was a potential N-glycosylation site, the humanized SE5 was modified with N53 S/T57S mutation, and the Fc region of SE5 (SEQ ID NO: 42) was modified with M135Y, S137T, and/or T139E (YTE), to enhance the affinity of the antibody molecules for the neonatal receptor FcRn under acidic conditions, thereby prolonging the half life of the antibody molecules in the body and extending the dosing period. The modified humanized molecules were referred to as SE5ss.

### Example 2 Detection on the blocking activity of the antigen binding protein of the present application (mouse-derived monoclonal antibody)

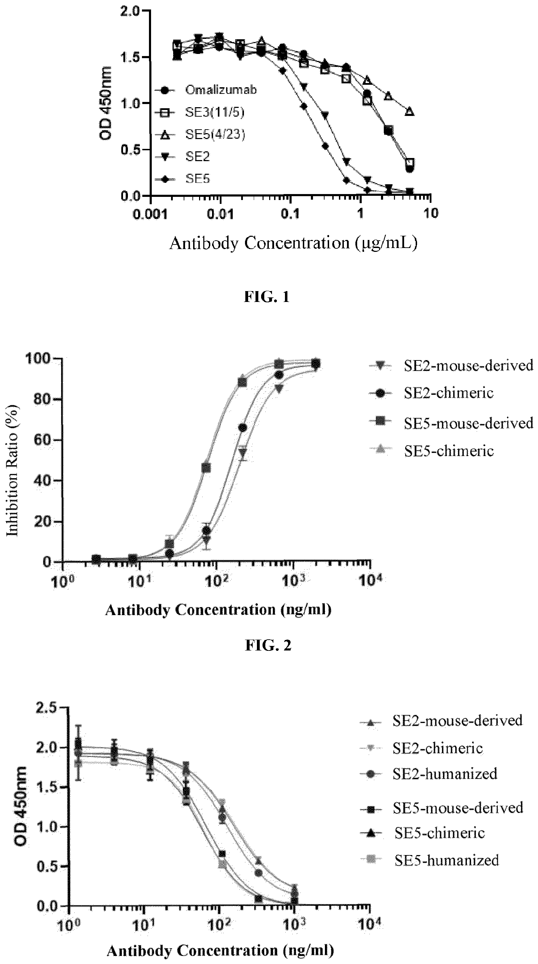

The blocking activity of the purified mouse-derived monoclonal antibody to block the binding of human IgE-Fc to its receptor FcεRIa was detected by an ELISA method. The method specifically included diluting the recombinantly expressed human FcεRIa-Fc protein (with its amino acid sequence as set forth in SEQ ID NO: 51) with PBS to a final concentration of 2.5 µg/mL, coating the ELISA plates (purchased from Corning, Product code: 42592) at 100 µL per well, and keeping at 4°C overnight. The next day, the coating solution was discarded. The wells were blocked with 2.5% skimmed milk dissolved in PBS for 1 hour and washed with PBS containing 0.05% Tween-20. A premixed solution containing 1 µg/mL of biotin-labelled human IgE-Fc (with its amino acid sequence as set forth in SEQ ID NO: 52) and gradiently diluted mouse-derived or control antibodies that has been pre-mixed for 10 min was added, in which the mouse-derived antibodies prepared in Example 1 or the control antibody Omalizumab (purchased from Novartis, Product code: Xolair) were diluted starting from 5 µg/mL at a 2-fold gradient (i.e., 5 µg/mL, 2.5 µg/mL, 1.25 µg/mL, 0.625 µg/mL, till 0.002441 µg/mL), and incubated at room temperature for 1 hour. After discarding the supernatant, the wells were washed with PBS containing 0.05% Tween-20 and diluted with HRP-labelled streptavidin (purchased from Sangon Biotech, Product code: D 111 054-000 1) at 1:1000 for detection. The detection results were shown in FIG. 1, showing that the mouse-derived monoclonal antibodies obtained from purification could block the ability of human IgE-Fc to bind its receptor FcεRIa to different extent, and SE2 and SE5 both had stronger blocking activity compared with the control antibody Omalizumab.

### Example 3 Detection on the blocking activity of the antigen binding protein of the present application (chimeric antibody)

To further verify the activity of the recombinantly expressed chimeric monoclonal antibody obtained from cloning, the blocking activity of the purified chimeric antibody to block the binding of human IgE-Fc to its receptor FcεRIa was detected by the ELISA method similar to that in Example 2. The method specifically included diluting the recombinantly expressed human FcεRIa-Fc protein (with its amino acid sequence as set forth in SEQ ID NO: 51) with PBS to a final concentration of 1 µg/mL, coating the ELISA plates (purchased from Corning, Product code: 42592) at 100 µL per well, and keeping at 4°C overnight. The next day, the coating solution was discarded. The wells were blocked with 2.5% skimmed milk dissolved in PBS for 1 hour and washed with PBS containing 0.05% Tween-20. A premixed solution containing 100 ng/mL of biotin-labelled human IgE-Fc (with its amino acid sequence as set forth in SEQ ID NO: 52) and gradiently diluted chimeric antibodies or mouse-derived antibodies that has been pre-mixed for 10 min was added, in which the chimeric antibodies or mouse-derived antibodies were diluted starting from 2 µg/mL at a 3-fold gradient (i.e., 2 µg/mL, 666.67 ng/mL, 222.22 ng/mL, 74.07 ng/mL, 24.69 ng/mL, 8.23 ng/mL, 2.74 ng/mL, till 0 ng/mL), and incubated at room temperature for 1 hour. After discarding the supernatant, the wells were washed with PBS containing 0.05% Tween-20 and diluted with HRP-labelled streptavidin (purchased from Sangon Biotech, Product code: D111054-0001) at 1:1000 for detection. The inhibition ratio of different antibodies at different concentrations was calculated following the formula below: Inhibition ratio at antibody concentration of X (%) = (OD₄₅₀ readout at antibody concentration of 0 - OD₄₅₀ readout at antibody concentration of X) / OD₄₅₀ readout at antibody concentration of 0 × 100. The detection results were shown in FIG. 2, showing that the chimeric recombinant monoclonal antibodies constructed from the sequences of the mouse-derived monoclonal variable regions obtained from cloning had a blocking activity close to that of the hybridoma purified mouse-derived parent antibodies.

### Example 4 Detection on the blocking activity of the antigen binding protein of the present application (anti-human IgE humanized antibody)

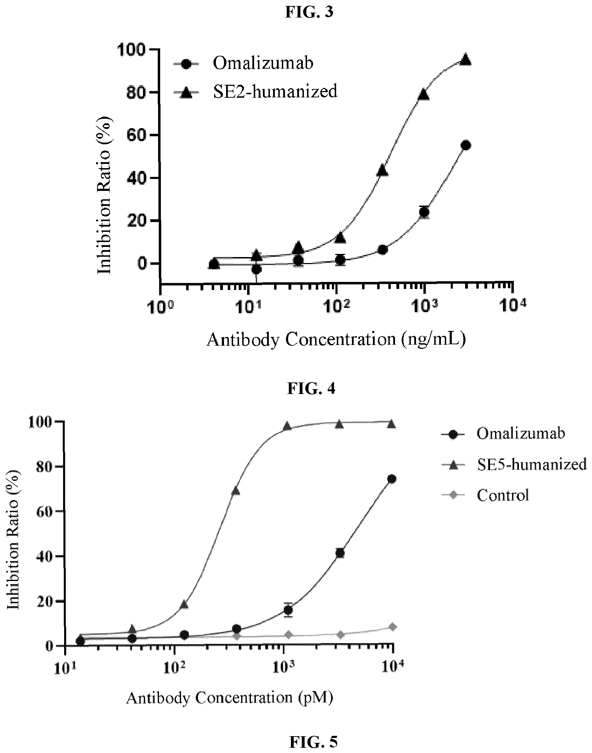

1) The blocking activity of the purified mouse-derived antibody, the chimeric antibody and the humanized antibody to block the binding of human IgE-Fc to its receptor FcεRIa was detected by the ELISA method similar to that in Example 2. The method specifically included diluting the recombinantly expressed human FcεRIa-Fc protein (with its amino acid sequence as set forth in SEQ ID NO: 51) with PBS to a final concentration of 1 µg/mL, coating the ELISA plates (purchased from Corning, Product code: 42592) at 100 µL per well, and keeping at 4°C overnight. The next day, the coating solution was discarded. The wells were blocked with 2.5% skimmed milk dissolved in PBS for 1 hour and washed with PBS containing 0.05% Tween-20. A premixed solution containing 100 ng/mL of biotin-labelled human IgE-Fc (with its amino acid sequence as set forth in SEQ ID NO: 52) and the gradiently diluted antibodies that has been pre-mixed for 10 min was added, in which the antibodies were diluted starting from 1 µg/mL at a 3-fold gradient (i.e., 1 µg/mL, 333.33 ng/mL, 111.11 ng/mL, 37.04 ng/mL, 12.35 ng/mL, 4.12 ng/mL, 1.37 ng/mL, till 0 ng/mL), and incubated at room temperature for 1 hour. After discarding the supernatant, the wells were washed with PBS containing 0.05% Tween-20 and detected by using HRP-labelled streptavidin (purchased from Sangon Biotech, Product code: D111054-0001). The detection results were shown in FIGs. 3 to 5, showing that the recombinantly expressed humanized monoclones SE2 and SE5 had a blocking activity close to that of its corresponding chimeric antibodies and mouse-derived parent antibodies, respectively (FIG. 3).
2) The blocking activity of the SE2 humanized antibodies, the SE5 humanized antibodies and Omalizumab to block the binding of human IgE-Fc to its receptor FcεRIa was detected following the method in step 1 of this example. In the comparative experiment between the SE2 humanized antibodies and Omalizumab, the antibody concentration was diluted starting from 3 µg/mL at a 3-fold gradient (i.e., 3 µg/mL, 1 µg/mL, 333.33 ng/mL, 111.11 ng/mL, 37.04 ng/mL, 12.35 ng/mL, 4.12 ng/mL, 1.37 ng/mL, till 0 ng/mL); and in the comparative experiment between the SE5 humanized antibodies and Omalizumab, the antibody concentration was diluted starting from 10 nM at a 3-fold gradient (i.e., 10 nM, 3.33 nM, 1.11 nM, 370.37 pM, 123.46 pM, 41.15 pM, 13.72 pM, till 0 pM). A control antibody was included in the SE5 comparative experiment (i.e., the control in FIG. 5, an isotype her2 antibody Pertuzumab, with its sequence derived from the IMGT antibody library).

The inhibition ratio of different antibodies at different concentrations was calculated following the formula below: Inhibition ratio at antibody concentration of X (%) = (OD₄₅₀ readout at antibody concentration of 0 - OD₄₅₀ readout at antibody concentration of X) / OD₄₅₀ readout at antibody concentration of 0 × 100. The results were shown in FIGs. 4 to 5, showing that both the SE2 humanized antibodies and the SE5 humanized antibodies had stronger blocking activity than that of the control antibody Omalizumab, where the blocking IC₅₀ values for SE2 and Omalizumab were about 416.7 ng/mL and 2110 ng/mL, respectively; and the blocking IC₅₀ values for SE5 and Omalizumab were about 266.2 pM and 4996 pM, respectively.

### Example 5 Detection on the affinity of the antigen binding protein of the present application (anti-human IgE humanized antibody)

The equilibrium affinity of humanized antibodies and human IgE was detected by MesoScale Discovery-Solution Equilibrium Titration (MSD-SET), the measurement method being described in Estep P. et, al., MAbs, 2013. 5(2): p. 270-8. The solution equilibrium titration (SET) was performed in PBS + 0.1% of IgG-free BSA buffer (PBSF), where the antigen human IgE (purchased from: abcam, Product code: ab65866) was kept constant at 10-100 pM and incubated with 3-fold serial dilutions of humanized antibodies starting from 0.1-100 nM. Specifically, for determination against SE2 and SE5, the antigen human IgE was kept constant at 13.33 pM, and the humanized antibodies SE2 and SE5 were 3-fold serially diluted from 166.67 pM to 0.0085 pM; while for determination against Omalizumab, the antigen human IgE was kept constant at 133.3 pM, and Omalizumab were 3-fold serially diluted from 66.67 nM to 1.13 pM. 20 nM of antibodies diluted in PBS were coated onto standard binding MSD-ECL plates (purchased from MSD, Product code: L15XA-3) at 4°C overnight. The MSD-ECL plates were blocked with 1% of Casein for 30 min while shaking at 700 rpm, and then washed with washing buffer (PBS+0.05% Tween 20) three times. The samples incubated with 2-fold serial dilutions of antigen human IgE and humanized antibodies were added into the MSD-ECL plates, incubated for 150 s while shaking at 700 rpm, and then washed once. Into the MSD-ECL plates was added PBSF containing 250 ng/mL of sulfo-labelled streptavidin, and 5 min later, the MSD-ECL plates were washed with the washing buffer (PBS + 0.05% Tween 20) three times. 1 × reading buffer T (purchased from MSD, Product code: R92TC-1) was added to read on an MSD Sector Imager instrument (Model: MESO QuickPlex SQ 120). In a Prism-GraphPad6 software, the percentage of free antigens was plotted as a function of titrated antibodies and fitted into a quadratic equation to extract the KD values. The measurement results were shown in Table 1 below.

**Table 1 Equilibrium affinity of humanized antibodies and the control antibody to human IgE as detected by MSD-SET method**

| **Name** | **KD (pM)** |
|---|---|
| Humanized antibody SE2 | 10.880 |
| Humanized antibody SE5 | 9.995 |
| Omalizumab | 2489 |

As can be known from the results shown in Table 1, the equilibrium affinities of humanized recombinant monoclonal antibodies SE2 and SE5 for binding to human IgE were about 10.880 pM and 9.995 pM, respectively, while the equilibrium affinity of the control antibody Omalizumab for binding to human IgE was about 2489 pM, with a significant improvement.

### Example 6 Detection on the affinity of the antigen binding protein of the present application (modified anti-human IgE humanized antibody)

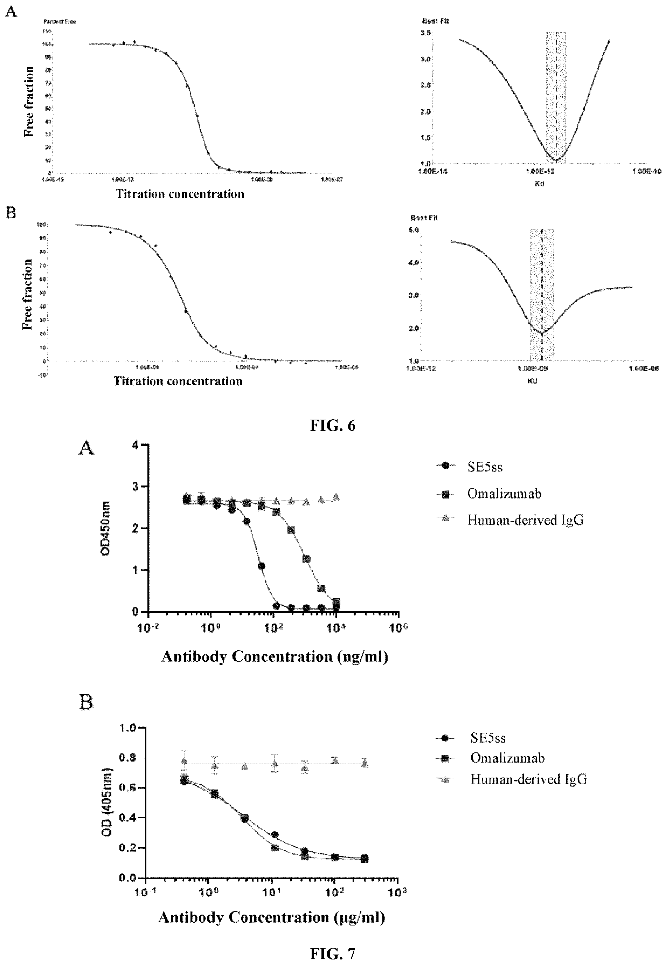

The equilibrium affinities of humanized antibodies and the control antibody (Omalizumab) to human IgE (derived from U266 cells (ATCC), see Mol Immunol. 1986, 23(2): 159-67 for its preparation method) were detected by using KinExA. The solution equilibrium titration (SET) was performed in PBS + 0.1% IgG-free BSA buffer (PBSF), where the antigen human IgE-Biotin (biotin-labelled human IgE, see the instruction of Thermofisher EZ-Link^{™} Sulfo-NHS-LC-Biotinylation Kit for its preparation method) was kept constant at 20 pM-5 nM and incubated together with 2 nM to 1 µM of 2-fold serially dilutions of antibodies. Specifically, for determination against the humanized antibody SE5ss, the antigen human IgE-Biotin was kept constant at 20 pM, the humanized antibody SE5ss was 2-fold serially diluted from 2 nM to 30.52 fM, and the mixed samples were incubated at room temperature for 30 hours; for determination against the control antibody Omalizumab, the antigen human IgE-Biotin was kept constant at 5 nM, Omalizumab was 2-fold serially diluted from 1 µM to 15.26 pM, and the mixed samples were incubated at room temperature for 20 hours. The samples incubated with 2-fold serial dilutions of antigen human IgE-Biotin and antibodies flew through the antibody-coated beads in the KinExA instrument (Model: 4000), and the free antigen human IgE-Biotin in the samples which had not been bound to the antibodies would bind to the antibodies coated on the beads. The fluorescence-conjugated secondary antibodies Alexa Fluor 647 Streptavidin continued to flow through the beads, and the instrument detected the fluorescence signals on the beads and fitted them by KinExA software to obtain the KD values. The measurement results can be seen in FIGs. 6A-B. The experiment results revealed that, the equilibrium affinities of the humanized recombinant monoclonal antibody SE5ss prepared in Example 1 and Omalizumab for binding to human IgE were about 2.08 pM and 1.79 nM, respectively, and the affinity of SE5ss was much higher than that of Omalizumab.

### Example 7 Detection on the blocking activity of the antigen binding protein of the present application (modified anti-human IgE humanized antibody)

### (1) Blocking activity of the antigen binding protein of the present application to block the binding of human-derived IgE to its receptor FcεRIa

The blocking activity of the SE5ss monoclonal antibody to block the binding of human-derived IgE to its receptor FcεRIa was detected by the ELISA method. The method specifically included diluting the recombinantly expressed human FcεRIa-Fc protein with PBS to 1 µg/mL, coating the ELISA plates (Corning, Product code: 42592) at 100 µL per well, and incubating at 4°C overnight. The next day, the coating solution was discarded. The wells were blocked with PBS containing 2.5% skimmed milk at 25°C for 1 hour and then washed with PBST (PBS + 0.05% Tween-20) three times. A premixed solution containing 100 ng/mL of human-derived IgE and gradiently diluted antibodies to be tested (SE5ss, Omalizumab and human-derived IgG) that has been pre-mixed for 1 hour was then added and incubated at 25°C for 1 hour, in which the antibodies to be tested were 3-fold serially diluted for 10 points, starting from 10 µg/mL to 0.17 ng/mL, plus a point of 0 µg/mL, and finally washed with PBST three times. Goat anti-human IgE antibodies (Invitrogen, Product code: A18795) diluted at 1:5000 and HRP-labelled rabbit-anti-goat IgG antibodies (Sangon, Product code: D110117-0100) diluted at 1:2000 were used for detection. The determined OD₄₅₀ values were analyzed using a four-parameter regression model to calculate the IC₅₀ of the antibodies. The results were shown in FIG. 7A, showing that both the SE5ss prepared in Example 1 and Omalizumab could effectively block the binding of human-derived IgE to the FcεRIα receptor at an IC₅₀ of 32.38 ng/mL and 1009 ng/mL, respectively, and the ability of SE5ss to block the binding of human-derived IgE to the FcεRIα receptor was much better than that of Omalizumab.

### (2) Blocking activity of the antigen binding protein of the present application to block the binding of human-derived IgE to its receptor, FcεRII (CD23) receptor

The blocking activity of SE5ss to block the binding of human-derived IgE to its receptor FcεRII (CD23) was detected by the ELISA method similar to that in step (1). The method specifically included diluting the recombinant human FcεRII (CD23, purchased from Sino Biological, Product code: 10261-H07H) protein with PBS to 1.5 µg/mL, and coating the ELISA plates (Corning, Product code: 42592) at 100 µL per well. The next day, the coating solution was discarded. The wells were blocked with 1% caseinat at room temperature for 2 hours. Biotin-labelled recombinantly expressed β-lactoglobulin-specific human IgE (bIgEk, Structure 15, 1413-1421, 2007) and β-lactoglobulin (sigma, Product code: L3908) were mixed in equal volume (at a molar ratio of approximately 1: 10) (the concentrations were both 60 µg/mL), incubated at 37°C for 1 hour and then further mixed with antibodies of gradiently concentrations, and incubated at 37°C for 1 hour. The antibodies to be tested (SE5ss, Omalizumab and human-derived IgG) were 3-fold serially diluted for 7 points, starting from 300 µg/mL to 0.41 µg/mL, plus a point of 0 µg/mL. The concentration of the bIgEk-Biotin and β-lactoglobulin complex was constantly 30 µg/mL. After then, the complex was washed with PBST three times. Streptavidin-HRP antibodies (purchased from Sangon, Product code: D110513-0100) diluted at 1:8000 were used for detection. The determined OD₄₅₀ values were analyzed using a four-parameter regression model to calculate the IC₅₀ of the antibodies. The results were shown in FIG. 7B, showing that both the SE5ss prepared in Example 1 and Omalizumab could effectively block the binding of human-derived IgE to the FcεRII (CD23) receptor at an IC₅₀ of 3.756 µg/mL and 3.444 µg/mL, respectively, and the ability of the two to block the binding of human-derived IgE to the FcεRII protein was similar.

### (3) Detection of the inhibitory activity of the antigen binding protein of the present application to inhibit the activation of human-derived IgE-mediated RBL-2H3-FcεRIα-NFATLuc cell reporter gene

Construction of RBL-2H3-FcεRIα-NFATLuc cell lines: to detect IgE-mediated cell activation and the activity of the antigen binding protein of the present application to block the cell level of IgE, NFAT-drived luciferase reporter gene cell lines expressing the human FcεRIα receptor were constructed on the basis of rat basophil RBL-2H3 cell lines (Cell Bank, Chinese Academy of Sciences), which were named as RBL-2H3-FcεRIα-NFATLuc. For the construction process, see Analytical and Bioanalytical Chemistry volume 412, pages 1901-1914 (2020); Allergy 2010; 65: 1266-1273; J Immunol July 1, 1996, 157 (1) 221-230.

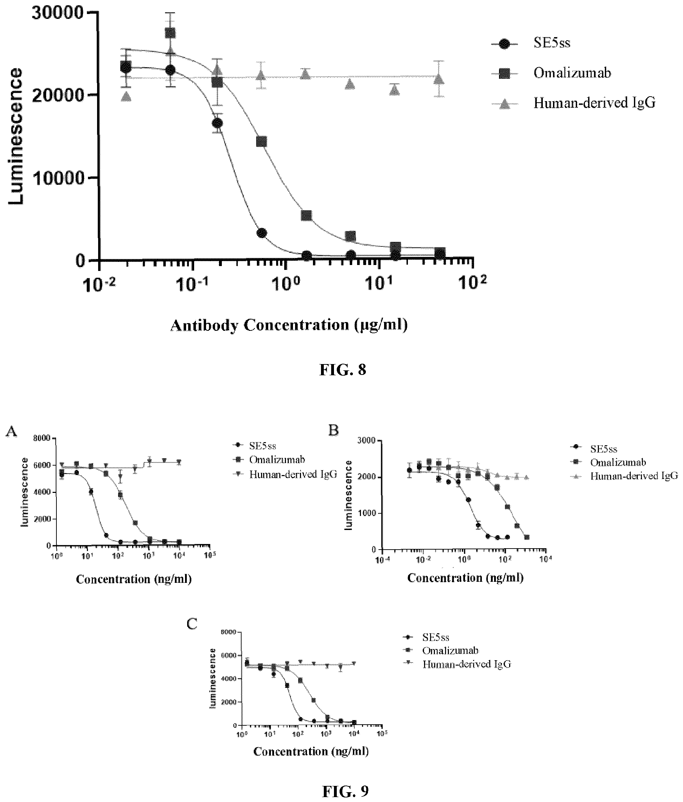

RBL-2H3-FcεRIα-NFATLuc cells at the logarithmic growth phase were plated on a 96-well flat plate at about 5E4 cells per well, in 50 µL medium. After then, a premixed solution containing human-derived IgE and gradient concentrations of antibodies to be tested (SE5ss, Omalizumab and human-derived IgG) was added and incubated in an incubator at 37°C and 5% CO₂ for around 20 hours. The final concentration of the human-derived IgE was constantly 1000 ng/mL, and the antibodies to be tested were 3-fold serially diluted for 8 points, starting from 45 µg/mL to 0.021 µg/mL, plus a point of 0 µg/mL. The next day, the plate was washed twice with PBS, a complete medium containing 10 µg/mL of anti-human IgE antibodies (purchased from Invitrogen, Product code: A18795) was added into each well and incubated in an incubator for around 3 hours. After completion, 100 µL of One-Lite TM (Vazyme) detection reagents were added into each well, transferred into a 96-well white plate after waiting for about 5 min, and detected by using the luminescence detection module of the Microplate reader. The results were fitted with a four-parameter regression model into an inhibition curve, from which the IC₅₀ of the antibodies was calculated. Ther results were shown in FIG. 8, showing that both SE5ss and Omalizumab could inhibit the activation of human-derived IgE-mediated effector cell reporter gene, with the IC₅₀ being 0.2641 µg/mL and 0.6093 µg/mL, respectively, and the inhibiting ability of SE5ss was obviously stronger than Omalizumab.

### (4) Detection on the activity of the antigen binding protein of the present application to inhibit the activation of RBL-2H3-FcεRIα-NFATLuc cells induced by human serum allergic to different allergens

RBL-2H3-FcεRIα-NFATLuc cells at the logarithmic growth phase were plated on a 96-well flat plate at about 5E4 cells per well, in 50 µL medium. Gradient concentrations of antibodies to be tested (SE5ss, Omalizumab and human-derived IgG) and human sera of different allergens were sequentially added into the cells and incubated in an incubator at 37°C and 5% CO₂ for around 20 hours. Since the IgE content of specific allergens varied among sera, different concentrations of sera and antibodies were used. The human sera of dust mite allergy (PlasmaLab, Product code: PL25740) was used at a concentration of 1.5%, and the antibodies were 3-fold serially diluted for 9 points, starting from 10000 ng/mL to a final concentration of 5 ng/mL; the human sera of shrimp allergy (PlasmaLab, Product code: PL24819) was used at a concentration of 3%, Omalizumab and human-derived IgG were 3-fold diluted for 12 points, starting from 1200.00 ng/mL to a final concentration of 0.01 ng/mL, and SE5ss was 3-fold serially diluted for 11 points, starting from 133.33 ng/mL to a final concentration of 0.002 ng/mL; the human sera of walnut allergy (PlasmaLab, Product code: PL27530) was used at a concentration of 1.5%, and the antibodies were 3-fold serially diluted for 9 points, starting from 10000 ng/mL to a final concentration of 1.5 ng/mL; a point of 0 µg/mL was added as control for all the three groups. The next day, the plate was washed twice with PBS, a complete medium containing 10 µg/mL of corresponding allergens (dust mite allergen, purchased from Greerlabs, Product code: XPB82D3A25; shrimp allergen, purchased from Wolcavi, Product code: M110227; walnut allergen, purchased from Wolcavi, Product code: M110343) was added into each well and incubated in an incubator for around 3 hours. After completion, 100 µL of One-Lite^{™} detection reagents were added into each well, transferred into a 96-well white plate after waiting for about 5 min, and detected by using the luminescence detection module of the Microplate reader. The results were fitted with a four-parameter regression model into an inhibition curve, from which the IC₅₀ of the antibodies was calculated. The experiment results showed that both the SE5ss prepared in Example 1 and Omalizumab could inhibit the activation of various allergic sera-mediated RBL-2H3-FcεRIα-NFATLuc cell reporter gene, in which the IC₅₀ for inhibition of dust mite allergic serum were 19.38 ng/mL and 177.9 ng/mL, respectively (FIG. 9A); the IC₅₀ for inhibition of shrimp allergic serum were 1.917 ng/mL and 229.8 ng/mL, respectively (FIG. 9B); the IC₅₀ for inhibition of walnut allergic serum were 51.57 ng/mL and 264.2 ng/mL, respectively (FIG. 9C); and the inhibiting ability of SE5ss was obviously superior to Omalizumab.

### Example 8 Detection on the activity of the antigen binding protein of the present application (modified anti-human IgE humanized antibody) in animals

### (1) Evaluation of the pharmacodynamics of the antigen binding protein of the present application in a human-derived IgE reconstruction mouse model

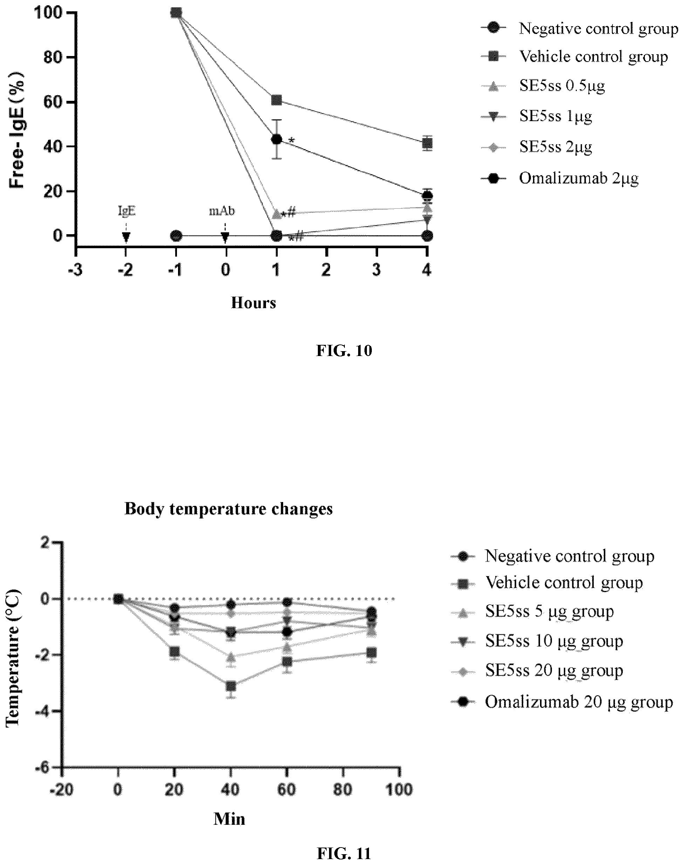

A human-derived IgE reconstruction model was established to evalute the inhibiting effect of SE5ss on the free IgE in mice. Balb/c mice (purchased from Beijing Vital River Laboratory Animal Technology Co., Ltd.) were adopted for test. 44 mice were randomly divided into a negative control group, a vehicle control group, an SE5ss 0.5 µg group, an SE5ss 1 µg group, an SE5ss 2 µg group and Omalizumab 2 µg group according to their gender and weight, where there were 4 mice in the negative control group, and 8 mice for each of the remaining groups. Each mouse was intravenously injected with 2 µg of human-derived IgE (derived from U266 culture purification, In house, Batch No. 5/20/29), where mice in the negative control group were intravenously injected with an equal volume of PBST (HyClone, Batch No. C520004-0001); and after 2 hours, intravenously injected with different doses of SE5ss and the positive drug Omalizumab (Novartis, Batch No. SPX27). Blood was collected from the mandibular vein of all the mice the day before administration, 1 hour before administration, 1 hour after administration, and 4 hours after administration, respectively, and the content of free IgE in the serum samples of mice were detected using MSD. The detection method was briefly described as below: the MSD plate was coated with FcεRIa-Fc (In house, Batch No.: 069-109-11), the samples to be tested were added, incubated at 37°C for 1 hour, and then washed with PBST three times; biotin-labelled mouse-anti-human IgE antibodies (In house, Clone# AE10-10) were added, incubated at 37°C for 1 hour, and then washed with PBST three times; and MSD sulfo-tag labeled streptavidin was used for detection (MSD, Batch No.: R32AD-1). SE5ss vehicle (In house, Batch No. 129-098-01) was used in the test as the negative control. The results were shown in FIG. 10, showing that both different concentrations of SE5ss prepared in Example 1 and Omalizumab could obviously inhibit free IgE in mice (P<0.05), and the inhibitory ability showed a dose-dependent effect, with the inhibitory ability of SE5ss at 0.5 µg per mouse on free IgE higher than that of Omalizumab at 2 µg per mouse (P<0.05).

### (2) Evaluation of the pharmacodynamics of the antigen binding protein of the present application in an exogenous reconstructed immediate hypersensitivity model

Human-FcεRIAtransgenic mice (purchased from Shanghai Model Organisms Center, Inc.) were used in the test to establish an exogenous reconstructed mouse model of immediate hypersensitivity. The mice were intraperitoneally injected with different doses of SE5ss, and the pharmacodynamics of SE5ss could be primarily evaluated according to the body temperature changes in mice. 51 human-FcεRIAtransgenic mice were randomly divided into a negative control group, a vehicle control group, a group of SE5ss at 5 µg per mouse, a group of SE5ss at 10 µg per mouse, a group of SE5ss at 20 µg per mouse, and a group of Omalizumab at 20 µg per mouse according to their gender and weight, where there were 6 mice in the negative control group, and 9 mice for each of the remaining groups. Animals in each group were intraperitoneally injected with different doses of SE5ss and Omalizumab (Novartis, Batch No. SPX27). 1 hour after administration, the animals in each group were intraperitoneally injected with human-derived IgE at 20 µg per mouse, and 24 hours after administration, anti-human IgE (ε-specific chain) goat antibodies (Sigma, Batch No. 16284-1 mg) were given by intraperitoneal injection at 20 µg per mouse to establish models of immediate hypersensitivity. The body temperature of mice was measured before and at 20 min, 40 min, 60 min and 90 min after the administration of anti-human IgE antibodies, respectively. SE5ss vehicle was used in the test as the negative control. The results were shown in FIG. 11, showing that after the administration of anti-human IgE antibodies, the body temperature of mice in the vehicle control group began to decrease, reached the lowest point at 40 min, and then rose back slowly. SE5ss in all dose groups could inhibit the decrease of body temperature in mice with a dose-dependent effect. The pharmacological effect of SE5ss at 10 µg per mouse was comparable to that of Omalizumab at 20 µg per mouse, and the body temperature of mice could be maintained within a normal range in the group of SE5ss at 20 µg per mouse.

### (3) Evaluation of the pharmacodynamics of the antigen binding protein of the present application in a Cynomolgus monkey IgE inhibiting model

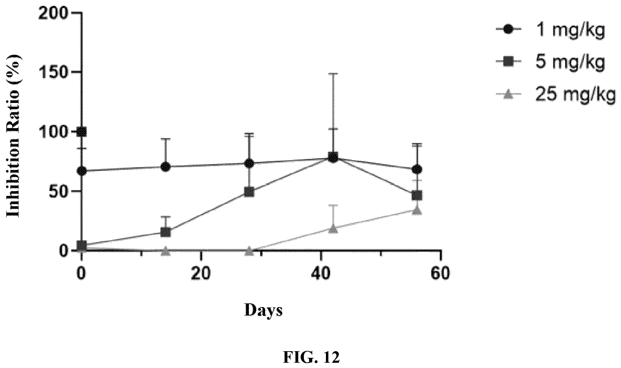

Different doses of SE5ss were intravenously injected in Cynomolgus monkey to evaluate its ability to inhibit the free IgE in Cynomolgus monkey. 18 Cynomolgus monkeys (Shanghai Institute of Materia Medica, Chinese Academy of Sciences) were randomly divided into 3 groups according to their gender and weight, with 3 Cynomolgus monkeys in each group for either gender, and were given a single subcutaneous injection of 1 mg/kg, 5 mg/kg, and 25 mg/kg of SE5ss, respectively. Serum samples were collected before administration, 1 hour after administration, and on days 14, 28, 42 and 56 after administration, and the content of free IgE was determined. The results were shown in FIG. 12, showing that different concentrations of SE5ss could obviously inhibit free IgE in Cynomolgus monkey, and the inhibiting ability showed a dose-dependent effect. In the 25 mg/kg group, the content of free IgE dropped to 2.4% of the pre-administration level at 1 hour after administration, and the free IgE were both below the lower limit of detection on days 14 and 28 and rose back to 19.1% and 34.8% of the baseline on days 42 and 56.

The foregoing detailed description is provided by way of explanation and examples and is not intended to limit the scope of the appended claims. Various changes of the embodiments currently set forth in this application are obvious to those of ordinary skills in the art and are reserved within the scope of the appended claims and their equivalents.

## Claims

1. An antigen binding protein, comprising at least one CDR of a heavy chain variable region and at least one CDR of a light chain variable region, wherein said heavy chain variable region comprises an amino acid sequence as set forth in any one of SEQ ID NOs: 73 or 74, and said light chain variable region comprises an amino acid sequence as set forth in any one of SEQ ID NOs: 75 or 76.

2. The antigen binding protein of claim 1, having one or more of the following properties:
1) capable of binding to IgE at a KD value of about 2.4×10⁻⁹ M or below;
2) capable of inhibiting the binding of IgE to its receptors FcεRIa and/or FcεRII.

3. The antigen binding protein of claim 2, wherein said IgE is human IgE.

4. The antigen binding protein of any one of claims 1-3, comprising a heavy chain HCDR3, wherein said HCDR3 comprises an amino acid sequence as set forth in SEQ ID NO: 3.

5. The antigen binding protein of any one of claims 1-4, further comprising a heavy chain HCDR2, wherein said HCDR2 comprises an amino acid sequence as set forth in SEQ ID NO: 77.

6. The antigen binding protein of claim 5, wherein said HCDR2 comprises an amino acid sequence as set forth in any one of SEQ ID NOs: 2 or 26.

7. The antigen binding protein of any one of claims 1-6, further comprising a heavy chain HCDR1, wherein said HCDR1 comprises an amino acid sequence as set forth in SEQ ID NO: 1.

8. The antigen binding protein of any one of claims 1-7, comprising heavy chain HCDR1, HCDR2 and HCDR3; wherein said HCDR1 comprises an amino acid sequence as set forth in SEQ ID NO: 1, said HCDR2 comprises an amino acid sequence as set forth in SEQ ID NO: 77, and said HCDR3 comprises an amino acid sequence as set forth in SEQ ID NO: 3.

9. The antigen binding protein of any one of claims 1-8, comprising heavy chain HCDR1, HCDR2 and HCDR3; wherein said HCDR1 comprises an amino acid sequence as set forth in SEQ ID NO: 1, said HCDR2 comprises an amino acid sequence as set forth in any one of SEQ ID NOs: 2 or 26, and said HCDR3 comprises an amino acid sequence as set forth in SEQ ID NO: 3.

10. The antigen binding protein of any one of claims 1-9, comprising a heavy chain H-FR1, wherein a C-terminal of said H-FR1 is connected to an N-terminal of said HCDR1 directly or indirectly, and said H-FR1 comprises an amino acid sequence as set forth in any one of SEQ ID NOs: 4 or 17.

11. The antigen binding protein of any one of claims 1-10, further comprising a heavy chain H-FR2, wherein said H-FR2 is located between said HCDR1 and said HCDR2, and said H-FR2 comprises an amino acid sequence as set forth in any one of SEQ ID NOs: 81, 5 or 27.

12. The antigen binding protein of claim 11, wherein said H-FR2 comprises an amino acid sequence as set forth in any one of SEQ ID NOs: 5, 18, 27 or 36.

13. The antigen binding protein of any one of claims 1-12, further comprising a heavy chain H-FR3, wherein said H-FR3 is located between said HCDR2 and said HCDR3, and said H-FR3 comprises an amino acid sequence as set forth in any one of SEQ ID NOs: 6 or 19.

14. The antigen binding protein of any one of claims 1-13, further comprising a heavy chain H-FR4, wherein an N-terminal of said H-FR4 is connected to a C-terminal of the HCDR3, and said H-FR4 comprises an amino acid sequence as set forth in any one of SEQ ID NOs: 7 or 20.

15. The antigen binding protein of any one of claims 1-14, comprising a heavy chain variable region VH, wherein said VH comprises an amino acid sequence as set forth in any one of SEQ ID NOs: 73 or 74.

16. The antigen binding protein of any one of claims 1-15, comprising a heavy chain variable region VH, wherein said VH comprises an amino acid sequence as set forth in any one of SEQ ID NOs: 8, 21, 28, 37 or 41.

17. The antigen binding protein of any one of claims 1-16, comprising a light chain LCDR3, wherein said LCDR3 comprises an amino acid sequence as set forth in SEQ ID NO: 79.

18. The antigen binding protein of claim 17, wherein said LCDR3 comprises an amino acid sequence as set forth in any one of SEQ ID NOs: 11 or 30.

19. The antigen binding protein of any one of claims 1-18, further comprising a light chain LCDR2, wherein said LCDR2 comprises an amino acid sequence as set forth in SEQ ID NO: 78.

20. The antigen binding protein of claim 19, wherein said LCDR2 comprises an amino acid sequence as set forth in any one of SEQ ID NOs: 10 or 29.

21. The antigen binding protein of any one of claims 1-20, further comprising a light chain LCDR1, wherein said LCDR1 comprises an amino acid sequence as set forth in SEQ ID NO: 9.

22. The antigen binding protein of any one of claims 1-21, comprising light chain LCDR1, LCDR2 and LCDR3; wherein said LCDR1 comprises an amino acid sequence as set forth in SEQ ID NO: 9, said LCDR2 comprises an amino acid sequence as set forth in SEQ ID NO: 78, and said LCDR3 comprises an amino acid sequence as set forth in SEQ ID NO: 79.

23. The antigen binding protein of any one of claims 1-22, comprising light chain LCDR1, LCDR2 and LCDR3; wherein said LCDR1 comprises an amino acid sequence as set forth in SEQ ID NO: 9, said LCDR2 comprises an amino acid sequence as set forth in any one of SEQ ID NOs: 10 or 29, and said LCDR3 comprises an amino acid sequence as set forth in any one of SEQ ID NOs: 11 or 30.

24. The antigen binding protein of any one of claims 1-23, comprising a light chain L-FR1, wherein a C-terminal of said L-FR1 is connected to an N-terminal of said LCDR1 directly or indirectly, and said L-FR1 comprises an amino acid sequence as set forth in any one of SEQ ID NOs: 31, 12 or 80.

25. The antigen binding protein of claim 24, wherein said L-FR1 comprises an amino acid sequence as set forth in any one of SEQ ID NOs: 12, 22, 31 or 38.

26. The antigen binding protein of any one of claims 1-25, further comprising a light chain L-FR2, wherein said L-FR2 is located between said LCDR1 and said LCDR2, and said L-FR2 comprises an amino acid sequence as set forth in any one of SEQ ID NOs: 13, 23 or 32.

27. The antigen binding protein of any one of claims 1-26, further comprising a light chain L-FR3, wherein said L-FR3 is located between said LCDR2 and said LCDR3, and said L-FR3 comprises an amino acid sequence as set forth in any one of SEQ ID NOs: 33, 14 or 82.

28. The antigen binding protein of claim 27, wherein said L-FR3 comprises an amino acid sequence as set forth in any one of SEQ ID NOs: 14, 24, 33 or 39.

29. The antigen binding protein of any one of claims 1-28, further comprising a light chain L-FR4, wherein an N-terminal of said L-FR4 is connected to a C-terminal of said LCDR3, and said L-FR4 comprises an amino acid sequence as set forth in any one of SEQ ID NOs: 34 or 15.

30. The antigen binding protein of any one of claims 1-29, comprising a light chain variable region VL, wherein said VL comprises an amino acid sequence as set forth in any one of SEQ ID NOs: 75 or 76.

31. The antigen binding protein of any one of claims 1-30, comprising a light chain variable region VL, wherein said VL comprises an amino acid sequence as set forth in any one of SEQ ID NOs: 16, 35, 25, or 40.

32. The antigen binding protein of any one of claims 1-31, comprising said HCDR1, HCDR2, HCDR3, LCDR1, LCDR2, and LCDR3, wherein said HCDR1 comprises an amino acid sequence as set forth in SEQ ID NO: 1, said HCDR2 comprises an amino acid sequence as set forth in SEQ ID NO: 77, said HCDR3 comprises an amino acid sequence as set forth in SEQ ID NO: 3, said LCDR1 comprises an amino acid sequence as set forth in SEQ ID NO: 9, said LCDR2 comprises an amino acid sequence as set forth in SEQ ID NO: 78, and said LCDR3 comprises an amino acid sequence as set forth in SEQ ID NO: 79.

33. The antigen binding protein of any one of claims 1-32, comprising said HCDR1, HCDR2, HCDR3, LCDR1, LCDR2, and LCDR3, wherein said HCDR1 comprises an amino acid sequence as set forth in SEQ ID NO: 1, said HCDR2 comprises an amino acid sequence as set forth in any one of SEQ ID NOs: 2 or 26, said HCDR3 comprises an amino acid sequence as set forth in SEQ ID NO: 3, said LCDR1 comprises an amino acid sequence as set forth in SEQ ID NO: 9, said LCDR2 comprises an amino acid sequence as set forth in any one of SEQ ID NOs: 10 or 29, and said LCDR3 comprises an amino acid sequence as set forth in any one of SEQ ID NOs: 11 or 30.

34. The antigen binding protein of any one of claims 1-33, comprising a heavy chain variable region VH and a light chain variable region VL, wherein said VH comprises an amino acid sequence as set forth in any one of SEQ ID NOs: 73 or 74, and said VL comprises an amino acid sequence as set forth in any one of SEQ ID NOs: 75 or 76.

35. The antigen binding protein of any one of claims 1-34, comprising a heavy chain variable region VH and a light chain variable region VL, wherein said VH comprises an amino acid sequence as set forth in any one of SEQ ID NOs: 8, 21, 28, 37 or 41, and said VL comprises an amino acid sequence as set forth in any one of SEQ ID NOs: 16, 35, 25, or 40.

36. The antigen binding protein of any one of claims 1-35, comprising an antibody or an antigen binding fragment thereof.

37. The antigen binding protein of claim 36, wherein said antigen binding fragment comprises Fab, Fab', F(ab)2, an Fv fragment, F(ab')2, scFv, di-scFv, and/or dAb.

38. The antigen binding protein of any one of claims 1-37, comprising a heavy chain constant region, wherein said heavy chain constant region is derived from human IgG.

39. The antigen binding protein of any one of claims 1-38, comprising a heavy chain constant region, wherein said heavy chain constant region is derived from human IgG1.

40. The antigen binding protein of any one of claims 1-39, comprising a heavy chain constant region, wherein said heavy chain constant region comprises an amino acid mutation below: M135Y, S137T and/or T139E, compared to an amino acid sequence as set forth in SEQ ID NO: 42.

41. The antigen binding protein of any one of claims 1-40, wherein said heavy chain constant region comprises an amino acid sequence as set forth in any one of SEQ ID NOs: 42 or 44.

42. The antigen binding protein of any one of claims 1-41, comprising a heavy chain, wherein said heavy chain comprises an amino acid sequence as set forth in any one of SEQ ID NOs: 45, 47 or 49.

43. The antigen binding protein of any one of claims 1-42, comprising a light chain constant region, wherein said light chain constant region comprises an amino acid sequence as set forth in SEQ ID NO: 43.

44. The antigen binding protein of any one of claims 1-43, comprising a light chain, wherein said light chain comprises an amino acid sequence as set forth in any one of SEQ ID NOs: 46 or 48.

45. The antigen binding protein of any one of claims 1-44, comprising a heavy chain and a light chain, wherein said heavy chain comprises an amino acid sequence as set forth in any one of SEQ ID NOs: 45, 47 or 49, and said light chain comprises an amino acid sequence as set forth in any one of SEQ ID NOs: 46 or 48.

46. A polypeptide, comprising the antigen binding protein of any one of claims 1-45.

47. An immunoconjugate, comprising the antigen binding protein of any one of claims 1-45.

48. An isolated nucleic acid molecule, encoding the antigen binding protein of any one of claims 1-45.

49. A vector, comprising the nucleic acid molecule of claim 48.

50. A cell, comprising and/or expressing the antigen binding protein of any one of claims 1-45, the polypeptide of claim 46, the immunoconjugate of claim 47, the nucleic acid molecule of claim 48 or the vector of claim 49.

51. A method for preparing the antigen binding protein of any one of claims 1-45, comprising culturing the cell of claim 50 under a condition enabling the expression of the antigen binding protein of any one of claims 1-45.

52. A pharmaceutical composition, comprising the antigen binding protein of any one of claims 1-45, the polypeptide of claim 46, the immunoconjugate of claim 47, the nucleic acid molecule of claim 48, the vector of claim 49, the cell of claim 50, and/or optionally a pharmaceutically acceptable carrier.

53. Use of the antigen binding protein of any one of claims 1-45, the polypeptide of claim 46, the immunoconjugate of claim 47, the nucleic acid molecule of claim 48, the vector of claim 49, the cell of claim 50 and/or the pharmaceutical composition of claim 52 in the preparation of a drug for preventing, alleviating, and/or treating an IgE-associated disease or disorder.

54. A method for preventing, alleviating, or treating an IgE-associated disease or disorder, comprising administering to a subject in need thereof the antigen binding protein of any one of claims 1-45, the polypeptide of claim 46, the immunoconjugate of claim 47, and/or the pharmaceutical composition of claim 52.

55. The antigen binding protein of any one of claims 1-45, the polypeptide of claim 46, the nucleic acid molecule of claim 48, the vector of claim 49, the cell of claim 50, the immunoconjugate of claim 47 and/or the pharmaceutical composition of claim 52, for use in preventing, alleviating, or treating an IgE-associated disease or disorder.

56. A method for detecting or determining IgE, comprising using the antigen binding protein of any one of claims 1-45 or the polypeptide of claim 46.

57. A kit for detecting or determining IgE, comprising the antigen binding protein of any one of claims 1-45 or the polypeptide of claim 46.
